(19) Europäisches Patentamt
European Patent Office
Office européen des brevets

(11) **EP 3 767 628 B1**

(12) **EUROPÄISCHE PATENTSCHRIFT**

(45) Veröffentlichungstag und Bekanntmachung des Hinweises auf die Patenterteilung:
**27.03.2024   Patentblatt 2024/13**

(21) Anmeldenummer: **20185117.7**

(22) Anmeldetag: **10.07.2020**

(51) Internationale Patentklassifikation (IPC):
***G16B 20/00*** (2019.01)   ***G16B 40/00*** (2019.01)

(52) Gemeinsame Patentklassifikation (CPC):
**G16B 20/00; G16B 40/00;** Y02A 90/10

(54) **SELEKTION VON ANTIKÖRPERN / ANTIKÖRPERFRAGMENTEN**

SELECTION OF ANTIBODIES / ANTIBODY FRAGMENTS

SÉLECTION D'ANTICORPS / DE FRAGMENTS D'ANTICORPS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2019   EP 19187005**

(43) Veröffentlichungstag der Anmeldung:
**20.01.2021   Patentblatt 2021/03**

(73) Patentinhaber: **Bayer Aktiengesellschaft 51371 Leverkusen (DE)**

(72) Erfinder:
• **Bender, Christian 50823 Köln (DE)**
• **Völker, Johanna 14109 Berlin (DE)**

(74) Vertreter: **BIP Patents c/o Bayer Intellectual Property GmbH Alfred-Nobel-Straße 50 40789 Monheim am Rhein (DE)**

(56) Entgegenhaltungen:
US-A1- 2013 178 370

• LINLING HE ET AL: "Hidden Lineage Complexity of Glycan-Dependent HIV-1 Broadly Neutralizing Antibodies Uncovered by Digital Panning and Native-Like gp140 Trimer", FRONTIERS IN IMMUNOLOGY, Bd. 8, 24. August 2017 (2017-08-24), XP055516159, DOI: 10.3389/fimmu.2017.01025
• J. KAPLINSKY ET AL: "Antibody repertoire deep sequencing reveals antigen-independent selection in maturing B cells", PROCEEDINGS OF THE NATIONAL ACADEMY OF SCIENCES, Bd. 111, Nr. 25, 24. Juni 2014 (2014-06-24), Seiten E2622-E2629, XP055662891, US ISSN: 0027-8424, DOI: 10.1073/pnas.1403278111
• NEHA CHAUDHARY ET AL: "Analyzing Immunoglobulin Repertoires", FRONTIERS IN IMMUNOLOGY, Bd. 9, 14. März 2018 (2018-03-14), XP055662897, DOI: 10.3389/fimmu.2018.00462

**Beschreibung**

Die vorliegende Erfindung befasst sich mit der Selektion von Antikörpern und/oder Antikörperfragmenten in einem Selektionsverfahren. Gegenstände der vorliegenden Erfindung sind ein Verfahren, ein System und ein Computerprogrammprodukt zur Erzeugung von Score-Werten für Paare von Genen, die die variablen Domänen von leichten und schweren Ketten von Antikörpern und/oder Antikörperfragmenten kodieren. Anhand der Score-Werte können Antikörper und/oder Antikörperfragmente selektiert werden.

Das humane Immunsystem bildet ein komplexes Netzwerk aus verschiedenen Organen, Zelltypen und Molekülen als Abwehrsystem gegen körperfremde Eindringlinge.

Antikörper, auch Immunglobuline genannt, sind Proteine aus der Klasse der Globuline, die in Wirbeltieren als Reaktion auf bestimmte Stoffe, so genannte Antigene, gebildet werden. Antikörper stehen im Dienste des Immunsystems; sie werden von einer Klasse weißer Blutzellen, den B-Lymphozyten, produziert.

Als Antigene wirken fast ausschließlich Makromoleküle oder an Partikel gebundene Moleküle, zum Beispiel Lipopolysaccharide an der Oberfläche von Bakterien. Ein bestimmtes Antigen induziert in der Regel die Bildung von nur wenigen bestimmten Antikörpern, die über spezifische, nicht-kovalente Bindungen zumeist nur diesen Fremdstoff erkennen.

Die spezifische Bindung von Antikörpern an die Antigene bildet einen wesentlichen Teil der Abwehr gegen die eingedrungenen Fremdstoffe.

Jeder Antikörper besteht aus zwei identischen schweren Ketten (engl.: *heavy chains,* H) und zwei identischen leichten Ketten (engl.: *light chains,* L), die durch kovalente Disulfidbrücken zu einer Ypsilon-förmigen Struktur miteinander verknüpft sind. Die leichten Ketten bestehen aus jeweils einer variablen und einer konstanten Domäne. Bezeichnet werden diese als $V_L$ und $C_L$. Die schweren Ketten hingegen haben jeweils eine variable und drei (IgG, IgA) bzw. vier (IgM, 1gE) konstante Domänen. Bezeichnet werden diese analog als $V_H$ und $C_H1$, $C_H2$, $C_H3$.

Die variablen Domänen einer leichten und einer schweren Kette bilden die Antigenbindungsstelle; diese sind daher von besonderem Interesse für therapeutische, immunologische und/oder diagnostische Zwecke. Große Bibliotheken von Antikörpern und/oder Antikörperfragmenten werden erstellt und ausgewertet, um diese z.B. in der Medizin zu nutzen.

Eine weitverbreitete Technik zum Generieren und Charakterisieren von Antikörperbibliotheken ist die "Phage Display"-Methode, bei der das jeweilige Protein von Interesse als Fusionspolypeptid auf einem Bakteriophagen-Hüllprotein exprimiert und durch Bindung an immobilisierten oder löslichen biotinylierten Liganden (Antigenen) selektiert werden kann. Ein Phage, der auf diese Weise konstruiert wurde, kann als kompakte genetische Einheit betrachtet werden, welche sowohl die phänotypischen als auch die genotypischen Eigenschaften in sich vereint hat. Die "Phage Display"-Technologie ist erfolgreich auf Antikörper, Antikörperfragmente, Enzyme, DNA-Bindungsproteine usw. angewendet worden.

Um z.B. ein Phagen-Display von Antikörper-Bibliotheken zu verwenden, werden zunächst die relevanten Zellen aus dem Organismus isoliert. Dabei handelt es sich um Plasmazellen, die insbesondere in Blut, Knochenmark und Lymphknoten zu finden sind. Aus diesen Zellen wird mRNA isoliert, die dann in cDNA transkribiert wird.

Mit Hilfe der Polymerase-Kettenreaktion (PCR) werden aus der cDNA die Gene der variablen Domänen der leichten ($V_L$) und schweren Kette ($V_H$) der Antikörper vervielfältigt.

Jeder Gensatz wird mit dem verkürzten Gen des Hüllproteins pIII (minor coat protein) des M13-Phagen in einem speziellen Phagemid-Vektor ligiert und *Escherichia coli* damit transformiert.

Dadurch exprimieren die *E. coli* Bakterien pIII-Fusionsproteine mit scFv-Fragmenten oder Fab-Antikörperfragmenten. Die Fusionsproteine werden durch ein Signalpeptid ins Periplasma transportiert, dort falten sie sich zu einem funktionalen scFv bzw. durch Disulfidbrücke verbundenem Fab-Fragment. Die Fv- bzw. Fab-Anteile bleiben zunächst über das pIII-Fragment in der inneren *E. coli*-Membran verankert und binden beim Abschluss des Zusammenbaus des Phagen an das Kapsid.

Über das Hüllprotein pIII, das normalerweise für die Infektion der Bakterien verantwortlich ist, wird nach Koinfektion mit einem M13-Helferphagen das funktionale Antikörperfragment beim Reifungsprozess neugebildeter Phagen in deren Außenhülle eingebaut. Gleichzeitig wird der Phagemid mit der zugehörigen genetischen Information für das entsprechende Antikörperfragment ins Innere der neugebildeten Phagen eingebaut. Jeder dieser rekombinanten Phagen hat also theoretisch ein anderes Antikörperfragment auf seiner Oberfläche und gleichzeitig die zugehörigen Gene ($V_L$ und $V_H$) in seinem Inneren, vergleichbar mit den Milliarden von B-Zellen im (menschlichen) Körper.

In einem sogenannten Biopanning können die "bindenden" Phagen über die auf der Oberfläche exponierten Antikörperfragmente durch Wechselwirkung mit fixierten Liganden (Antigenen) aus dem milliardenfachen Hintergrund der irrelevanten Phagen selektiert werden.

Üblicherweise werden beim Biopanning mehrere Selektionszyklen (Panning-Runden) durchlaufen. Üblicherweise wird dazu eine Phage-Display Bibliothek einem Substrat ausgesetzt, so dass die Bindung von einigen Phagen stattfinden kann. Unspezifisch bindende und schwach bindende Phagen werden abgewaschen. Nach dem Waschen noch bindende und damit spezifische Phagen werden anschließend abgelöst (eluiert). Die eluierten Phagen werden vermehrt und in weiteren Panning-Runden erneut dem Substrat ausgesetzt, bis sich eine Population von gut bindenden Phagen anreichert.

Am Ende des Selektionsprozesses können aus den isolierten Phagen die zugehörigen Antikörpergene einfach isoliert und sequenziert werden. Die Sequenzierung ergibt dann Auskunft über die Baupläne der Antiköper(fragmente). Weitere Verfahren zur Selektion von Antikörpern und/oder Antikörperfragmenten sind in der Literatur beschrieben (siehe z.B. Sai T. Reddy et al.: Monoclonal antibodies isolated without screening by $\alpha$n$\alpha$ylzing the variable-gene repertoire of plasma cells, Nature Biotechnology Vol. 28 No. 9, Sept. 2010, 965-971).

Eine Standard-Methode zur Sequenzbestimmung ist die Didesoxymethode nach Sanger (Kettenabbruch-Synthese). Die Länge der so sequenzierten DNA-Abschnitte (Reads) kann mehr als 1000 Basenpaare erreichen, dies ist jedoch mit hohen Kosten und Zeitaufwand verbunden. Eine Alternative stellt die Hochdurchsatz-Sequenzierung (engl.: *Next Generation Sequencing,* NGS) dar. Dank der Detektion von vielen parallel ablaufenden und räumlich getrennten Sequenzierungsreaktionen auf kleinster Fläche wird im Vergleich zum Standard schneller ein deutlich höherer Durchsatz bei kürzeren Read-Längen erreicht. NGS erreicht eine Größenordnung von $10^6$ Sequenzierungen. Damit ist es möglich, die Vielfalt und Qualität einer ganzen Bibliothek zu bestimmen.

In einem Biopanning-Selektionsverfahren enthält der letzte Pool trotz stetiger Anreicherung spezifisch bindender Antikörperfragmente noch immer eine Vielzahl an verschiedenen Antikörperfragmenten. Die Zahl ist zu groß, um alle Antiköperfragmente näher zu untersuchen; es muss eine Auswahl getroffen werden. Oftmals werden die Antikörperfragmente in eine Rangfolge gemäß der Zahl ihrer $V_H$-Gene im letzten Pool gebracht. Je mehr sich die Antikörperfragmente angereichert haben, desto höher ist in der Regel auch die Zahl ihrer $V_H$-Gene im letzten Pool.

Allerdings ist die Zahl der $V_H$-Gene im letzten Pool kein hinreichendes Kriterium für die Auswahl besonders interessanter und/oder lohnenswerter Antikörper.

US 2013/178370 A1 beschreibt die Identifizierung von Antikörperfragmenten, zum Beispiel mit einem Ober Phagen-Display Verfahren. Next-Generation-Sequencing wird zum Bestimmen der Gene verwendet. Paarungen von $V_L$ und $V_H$ Genen korrelieren mit der Reihenfolge der Häufigkeit der zugehörigen Proteine in einer Probe. Die Methode wird in der Analyse eines Anreicherungsverfahrens verwendet.

Auch US 2013/178370 A1 beschreibt kein hinreichendes Kriterium für die Auswahl besonders interessanter und/oder lohnenswerter Antikörper.

Es wäre daher wünschenswert, eine bessere Hilfestellung für die Identifizierung von erfolgsversprechenden Antikörperfragmenten zur Verfügung haben.

Diese Aufgabe wird durch die Gegenstände der unabhängigen Patentansprüche gelöst. Bevorzugte Ausführungsformen finden sich in den abhängigen Patentansprüchen, der vorliegenden Beschreibung und den Abbildungen.

Ein erster Gegenstand der vorliegenden Erfindung ist ein Verfahren umfassend die Schritte:

- Empfangen von Merkmalen zu $V_L$-Genen und $V_H$-Genen, wobei die $V_L$-Gene und $V_H$-Gene die variablen Domänen von leichten und schweren Ketten von Antiköpern und/oder Antikörperfragmente kodieren, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,

- Bilden von Merkmalvektoren für Paare von $V_L$-Genen und $V_H$-Genen in den Pools, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

  • Häufigkeit des $V_L$-Gens in dem jeweiligen Pool,
  • Häufigkeit des $V_H$-Gens in dem jeweiligen Pool,
  • Häufigkeit des $V_L$-Gens in dem vorangegangenen Pool,
  • Häufigkeit des $V_H$-Gens in dem vorangegangenen Pool,

- Ermitteln von Score-Werten für Paare von $V_L$-Genen und $V_H$-Genen auf Basis der Anreicherungsmuster, wobei die Score-Werte mit der jeweiligen Stärke der Anreicherung korrelieren,

- optional: Ermitteln einer Rangliste für Paare von $V_L$-Genen und $V_H$-Genen auf Basis der Score-Werte,

- Ausgeben der Score-Werte und/oder der Rangliste gegenüber einem Nutzer.

Ein weiterer Gegenstand der vorliegenden Erfindung ist ein System umfassend:

• eine Eingabeeinheit,
• eine Merkmalvektorerzeugungseinheit,
• eine Anreicherungsanalyseeinheit,
• eine Score-Wert-Berechnungseinheit,
• eine Ranking-Einheit und
• eine Ausgabeeinheit,

- wobei die Eingabeeinheit konfiguriert ist, Merkmale zu $V_L$-Genen und $V_H$-Genen, die variable Domänen von leichten und schweren Ketten von Antiköpern und/oder Antikörperfragmenten kodieren, zu erfassen, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,

- wobei die Merkmalvektorerzeugungseinheit konfiguriert ist, auf Basis der erfassten Merkmale Merkmalvektoren für Paare von $V_L$- und $V_H$- Genen in den Pools zu erzeugen, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

  - Häufigkeit des $V_L$-Gens in dem jeweiligen Pool,
  - Häufigkeit des $V_H$-Gens in dem jeweiligen Pool,
  - Häufigkeit des $V_L$-Gens in dem vorangegangenen Pool,
  - Häufigkeit des $V_H$-Gens in dem vorangegangenen Pool,
  - die absolute Differenz zwischen der Häufigkeit des $V_H$-Gens in dem jeweiligen Pool und der Häufigkeit des $V_L$-Gens in dem jeweiligen Pool,
  - die absolute Differenz zwischen der Häufigkeit des $V_H$-Gens in dem vorangegangenen Pool und der Häufigkeit des $V_L$-Gens in dem vorangegangenen Pool,

- wobei die Anreicherungsanalyseeinheit konfiguriert ist, Anreicherungsmuster in den Merkmalvektoren der Pools zu identifizieren, wobei Anreicherungsmuster zeigen, wie stark sich der Antikörper und/oder das Antikörperfragment mit dem jeweiligen $V_L$-Gen und $V_H$-Gen in dem Selektionsverfahren anreichern,

- wobei die Score-Wert-Berechnungseinheit konfiguriert ist, Score-Werte für Paare von $V_L$-Genen und $V_H$-Genen auf Basis der Anreicherungsmuster zu berechnen, wobei die Score-Werte mit der jeweiligen Stärke der Anreicherung korrelieren,

- wobei die Ranking-Einheit vorzugsweise konfiguriert ist, eine Rangliste für Paare von $V_L$-Genen und $V_H$-Genen auf Basis der Score-Werte zu berechnen,

- wobei die Ausgabeeinheit konfiguriert ist, die Score-Werte und/oder die Rangliste gegenüber einem Nutzer auszugeben.

Ein weiterer Gegenstand der vorliegenden Erfindung ist ein Computerprogrammprodukt umfassend einen Datenträger, und Programmcode, der auf dem Datenträger gespeichert ist, und der ein Computersystem, in dessen Arbeitsspeicher der Programmcode geladen ist, dazu veranlasst, die folgenden Schritte auszuführen:

- Empfangen von Merkmalen zu $V_L$-Genen und $V_H$-Genen, wobei die $V_L$-Gene und $V_H$-Gene die variablen Domänen von leichten und schweren Ketten von Antiköpern und/oder Antikörperfragmenten kodieren, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,

- Bilden von Merkmalvektoren für Paare von $V_L$-Genen und $V_H$-Genen in den Pools, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

  - Häufigkeit des $V_L$-Gens in dem jeweiligen Pool,
  - Häufigkeit des $V_H$-Gens in dem jeweiligen Pool,
  - Häufigkeit des $V_L$-Gens in dem vorangegangenen Pool,
  - Häufigkeit des $V_H$-Gens in dem vorangegangenen Pool,
  - die absolute Differenz zwischen der Häufigkeit des $V_H$-Gens in dem jeweiligen Pool und der Häufigkeit des $V_L$-Gens in dem jeweiligen Pool,
  - die absolute Differenz zwischen der Häufigkeit des $V_H$-Gens in dem vorangegangenen Pool und der Häufigkeit des $V_L$-Gens in dem vorangegangenen Pool,

- Identifizieren von Anreicherungsmustern in den Merkmalvektoren der Pools, wobei Anreicherungsmuster zeigen, wie stark sich der Antikörper und/oder das Antikörperfragment mit dem jeweiligen $V_L$-Gen und $V_H$-Gen in dem Selektionsverfahren anreichern,

- Ermitteln von Score-Werten für Paare von $V_L$-Genen und $V_H$-Genen auf Basis der Anreicherungsmuster, wobei die Score-Werte mit der jeweiligen Stärke der Anreicherung korrelieren,

- optional: Ermitteln einer Rangliste für Paare von $V_L$-Genen und $V_H$-Genen auf Basis der Score-Werte,

- Ausgeben der Score-Werte und/oder der Rangliste gegenüber einem Nutzer.

Die Erfindung wird nachstehend näher erläutert, ohne zwischen den Erfindungsgegenständen zu unterscheiden. Die nachfolgenden Erläuterungen sollen vielmehr für alle Erfindungsgegenstände in analoger Weise gelten, unabhängig davon, in welchem Kontext sie erfolgen.

Wenn in der vorliegenden Beschreibung oder in den Patentansprüchen Schritte in einer Reihenfolge genannt werden, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden können; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen dar.

Ein Ausgangspunkt für die vorliegende Erfindung ist eine Bibliothek von Antikörpern und/oder Antikörperfragmenten. Diese Antikörper und/oder Antikörperfragmente werden einem Selektionsverfahren zugeführt, um Antikörper und/oder Antikörperfragmente auf Basis ihrer phänotypischen Eigenschaften zu selektieren. Bei dem Selektionsverfahren kann es sich beispielsweise um ein Biopanning-Verfahren handeln.

Ein Selektionsverfahren im Sinn der vorliegenden Erfindung umfasst mehrere aufeinander folgende Selektionszyklen. Das Ergebnis eines jeden Selektionszyklus ist ein Pool mit Antikörpern und/oder Antikörperfragmenten. Das Ziel des Selektionsverfahrens ist eine Anreicherung von spezifisch bindenden Antikörpern und/oder Antikörperfragmenten. Das bedeutet, dass ein beliebiger Pool in dem Selektionszyklus eine höhere Konzentration an spezifisch bindenden Antikörpern und/oder Antikörperfragmenten aufweist als ein diesem Pool vorangegangener Pool.

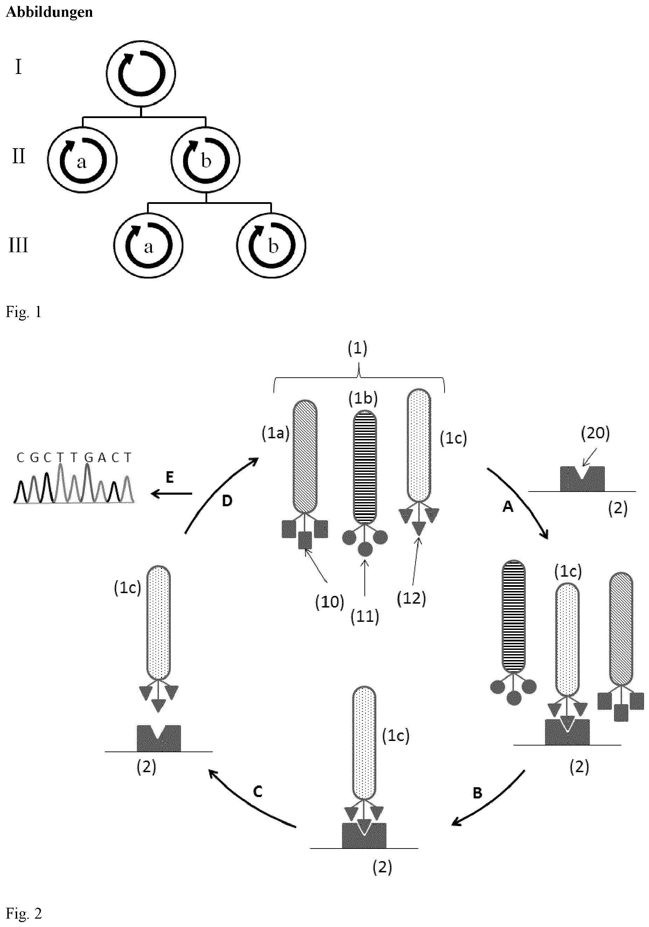

Fig. 1 zeigt ein Beispiel für eine Selektionshierarchie mit drei Ebenen (I, II, III). In der ersten Ebene (I) wird eine Bibliothek von Antikörpern und/oder Antikörperfragmenten einem ersten Selektionszyklus zugeführt. Das Ergebnis des ersten Selektionszyklus ist ein Pool selektierter Antikörper und/oder Antikörperfragmente. Die selektierten Antikörper und/oder Antikörperfragmente werden in der zweiten Ebene (11) zwei Selektionszyklen zugeführt (a und b). In der Regel handelt es sich bei den Selektionszyklen in der zweiten Ebene um verschiedene Selektionszyklen. Die Ergebnisse der Selektionen in der zweiten Ebene sind wiederum zwei Pools von selektierten Antikörpern und/oder Antikörperfragmenten. Der aus dem rechten Ast (IIb) der zweiten Ebene resultierende Pool an Antikörpern und/oder Antikörperfragmenten wird in der dritten Ebene (III) erneut zwei Selektionszyklen (a und b) ausgesetzt, die wiederum zwei Pools zum Ergebnis haben. In der Regel werden die Antikörper und/oder Antikörperfragmente beim Durchlaufen einer Selektionshierarchie von oben nach unten (z.B. von der ersten Ebene über die zweite Ebene zur dritten Ebene) einem zunehmenden Selektionsdruck ausgesetzt. Wird eine Selektionshierarchie von oben nach unten (I → IIb → IIIb) durchlaufen, nimmt die Diversität der Antikörper und/oder Antikörperfragmente in den jeweiligen Pools ab.

Es ist denkbar, dass in den Selektionszyklen verschiedene Targets eingesetzt werden. So ist es beispielsweise üblich, bei der Suche nach therapeutisch wirksamen Antikörpern oder bei der Suche nach Antikörpern für diagnostische Zwecke die Bindung der Antikörper nicht nur in Bezug auf ein humanes Target zu untersuchen, sondern auch in Bezug auf ein murines Target. Dementsprechend weist ein Selektionsverfahren oftmals mindestens zwei Äste auf, wobei die Antikörpern in einem Ast einem zunehmenden Selektionsdruck gegenüber einem ersten Target (z.B. einem Maus-Target) und in dem zweiten Ast einem zunehmenden Selektionsdruck gegenüber einem zweiten Target (z.B. einem humanen Target) ausgesetzt werden (siehe z.B.: WO0220822A2).

Vorzugsweise umfasst das Selektionsverfahren mindestens zwei, vorzugsweise mindestens 3, noch mehr bevorzugt mindestens 4, am meisten bevorzugt mindestens 5 Selektionsebenen.

Im Anschluss an einen Selektionszyklus werden Gene der selektierten Antikörper und/oder Antikörperfragmente üblicherweise sequenziert, um sie zu identifizieren.

Bei der Sequenzierung werden die $V_L$-Gene, die die variablen Domänen von leichten Ketten der Antikörper und/oder Antikörperfragmente kodieren, und die $V_H$-Gene, die die variablen Domänen der schweren Ketten der Antikörper und/oder Antikörperfragmente kodieren, bestimmt. Gleichzeitig werden ihre Häufigkeiten (engl.: *counts*) in dem jeweiligen Pool bestimmt.

Es ist denkbar, dass ein solcher Sequenzierungsschritt nach jedem Selektionszyklus durchgeführt wird. Denkbar ist auch, dass ein solcher Sequenzierungsschritt erst nach Durchlaufen einer Hierarchie von mehreren Zyklen durchgeführt wird. Es ist denkbar, dass zunächst eine gewisse Anreicherung spezifisch bindender Antikörper und/oder Antikörperfragmente erreicht werden soll, bevor eine Charakterisierung durch Sequenzierung stattfindet.

In einem weiteren Schritt werden Merkmale zu den $V_L$- und $V_H$-Genen in den jeweiligen Pools erfasst/ermittelt und Merkmalvektoren für Paare von $V_L$-Genen und $V_H$-Genen erzeugt, wobei das $V_L$-Gen und das $V_H$-Gen eines jeden Paares variable Domänen kodieren, die zum selben Antikörper und/oder Antikörperfragment gehören.

Generell gilt, dass ein Merkmalvektor (engl.: *feature vector*) die (vorzugsweise numerisch) parametrisierbaren Eigenschaften (Merkmale) eines *Objekts* in vektorieller Weise zusammenfasst. Verschiedene, für das *Objekt* charakteristische Merkmale bilden die verschiedenen Dimensionen dieses Vektors. Die Gesamtheit der möglichen Merkmalvektoren nennt man den Merkmalsraum. Merkmalvektoren erleichtern beispielsweise eine automatische Klassifizierung, da sie die zu klassifizierenden Eigenschaften stark reduzieren.

Das *Objekt* ist im vorliegenden Fall ein Paar eines V$_L$-Gens und eines V$_H$-Gens in einem Pool, wobei das V$_L$-Gen und das V$_H$-Gen variable Domänen kodieren, die zum selben Antikörper und/oder Antikörperfragment gehören.

Eine Möglichkeit zur Identifizierung von Paaren von V$_L$-Genen und V$_H$-Genen, bei denen das V$_L$-Gen und das V$_H$-Gen variable Domänen kodieren, die zum selben Antikörper und/oder Antikörperfragment gehören, ist in der Internationalen Patentanmeldung mit der Veröffentlichungsnummer WO2019206729 beschrieben.

Da die genannte Patentanmeldung am Prioritätstag des vorliegenden Schutzrechts noch nicht offengelegt war, sind die wesentlichen Aspekte zur Identifizierung von Paaren von V$_L$-Genen und V$_H$-Genen, bei denen das V$_L$-Gen und das V$_H$-Gen variable Domänen kodieren, die zum selben Antikörper und/oder Antikörperfragment gehören, im letzten Abschnitt der vorliegenden Beschreibung aufgeführt.

Ein Merkmalvektor charakterisiert ein Paar eines V$_L$-Gens und eines V$_H$-Gens in einem Pool. Für dasselbe Paar in einem anderen Pool (z.B. dem vorangegangenen Pool) wird ein separater Merkmalvektor erzeugt. Ebenso wird für ein anderes Paar in demselben Pool auch ein separater Merkmalvektor erzeugt. In einen Merkmalvektor für ein Paar in einem Pool fließen erfindungsgemäß nicht nur Informationen zu dem Paar in dem Pool sondern beispielsweise auch Informationen zu demselben Paar in dem vorangegangenen Pool ein.

Ein Merkmalvektor für ein Paar eines V$_L$-Gens und eines V$_H$-Gens für einen Pool umfasst zumindest die folgenden Merkmale:

- Häufigkeit des V$_L$-Gens in dem jeweiligen Pool (vorzugsweise in Form der absoluten Zahl VL der V$_L$-Gene in dem Pool oder in normalisierter Form),
- Häufigkeit des V$_H$-Gens in dem jeweiligen Pool (vorzugsweise in Form der absoluten Zahl VH der V$_H$-Gene in dem Pool oder in normalisierter Form),
- Häufigkeit des V$_L$-Gens in dem vorangegangenen Pool (vorzugsweise in Form der absoluten Zahl prevVL des V$_L$-Gens in dem vorangegangenen Pool oder in normalisierter Form)
- Häufigkeit des V$_H$-Gens in dem vorangegangenen Pool (vorzugsweise in Form der absoluten Zahl prevVH des V$_H$-Gens in dem vorangegangenen Pool oder in normalisierter Form)
- die absolute Differenz (diff) zwischen der Häufigkeit des V$_H$-Gens in dem jeweiligen Pool und der Häufigkeit des V$_L$-Gens in dem jeweiligen Pool (diff = |VH - VL|)
- die absolute Differenz (prevDiff) zwischen der Häufigkeit des V$_H$-Gens in dem vorangegangenen Pool und der Häufigkeit des V$_L$-Gens in dem vorangegangenen Pool (prevDiff = |prevVH - prevVL|).

Wichtige Merkmale zu V$_L$- und V$_H$-Genen in einem Pool sind ihre Häufigkeiten, mit denen sie in dem jeweiligen Pool auftreten (vorzugsweise in Form der absoluten Zahlen VH und VL oder in normalisierter Form). Die Häufigkeiten werden vorzugsweise per Next-Generation-Sequencing-Verfahren erfasst, indem die fragmentierte genetische Information von V$_H$ und V$_L$ Sequenzen mit Hilfe gepaarter Erkennungssequenzen (Primer-Paare) zu vollständigen V$_H$ und V$_L$ Ketten (oder Teilen davon) assembliert werden. Die eindeutigen Sequenzen werden dann gezählt und in Form von DNA-Counts ausgegeben.

Die Normalisierung der Häufigkeiten kann beispielsweise auf Basis der folgenden Formeln erfolgen:

$$rpmVH = (VH \,/\, sumVH) * 1.000.000$$

$$rpmVL = (VL \,/\, sumVL) * 1.000.000$$

wobei rpmVH die normalisierte Häufigkeit der V$_H$-Gene in dem betrachteten Pool ist,

wobei rpmVL die normalisierte Häufigkeit der V$_L$-Gene in dem betrachteten Pool ist,

wobei sumVH die Summe der absoluten Häufigkeiten VH aller V$_H$-Gene in dem betrachteten Pool ist,

wobei sumVL die Summe der absoluten Häufigkeiten VL aller V$_L$-Gene in dem betrachteten Pool ist.

Weitere wichtige Merkmale sind die Häufigkeit des V$_L$-Gens in dem vorangegangenen Pool und die Häufigkeit des V$_H$-Gens in dem vorangegangenen Pool. Unter dem vorangegangenen Pool wird vorzugsweise derjenige Pool verstanden, der in der Selektionshierarchie eine Ebene oberhalb, d.h. in der Reihenfolge der durchlaufenen Selektionszyklen unmittelbar vor einem betrachteten Pool liegt. Die aus dem vorangegangenen Pool isolierten Antikörper / Antikörperfragmente werden dem Selektionszyklus zugeführt, dessen Ergebnis der betrachtete Pool ist. Auch die Häufigkeit der V$_L$-Gene und der V$_H$-Gene in dem vorangegangenen Pool können in Form absoluter Häufigkeiten oder in normalisierter Form in einem Merkmalvektor verwendet werden.

Weitere wichtige Merkmale sind die absolute Differenz (diff) zwischen der Häufigkeit des V$_H$-Gens in dem betrachteten Pool und der Häufigkeit des V$_L$-Gens in dem betrachteten Pool (diff = |VH - VL|) sowie die absolute Differenz (prevDiff) zwischen der Häufigkeit des V$_H$-Gens in dem vorangegangenen Pool und der Häufigkeit des V$_L$-Gens in dem vorangegangenen Pool (prevDiff = |prevVH - prevVL|).

Ferner können weitere Merkmale in den Merkmalvektor aufgenommen werden, wie zum Beispiel:

- die absolute Zahl (Häufigkeit) unterschiedlicher

$V_H$-Gene in dem betrachteten Pool: numVH

- die absolute Zahl (Häufigkeit) unterschiedlicher $V_L$-Gene in dem betrachteten Pool: numVL

- die absolute Zahl (Häufigkeit) desjenigen $V_H$-Gens, das in dem betrachteten Pool am häufigsten vorhanden ist: maxVH

- die absolute Zahl (Häufigkeit) desjenigen $V_L$-Gens, das in dem betrachteten Pool am häufigsten vorhanden ist: maxVL

- die relative Häufigkeit des $V_H$-Gens in dem betrachteten Pool (bezogen auf die Zahl des am häufigsten in dem betrachteten Pool auftretenden $V_H$-Gens): relVH = VH/maxVH

- die relative Häufigkeit des $V_L$-Gens in dem betrachteten Pool (bezogen auf die Zahl des am häufigsten in dem betrachteten Pool auftretenden $V_L$-Gens): relVL = VL/maxVL

- der relative Unterschied (relativer Abstand) zwischen der Häufigkeit des $V_H$-Gens in dem betrachteten Pool und der Häufigkeit des $V_L$-Gens in dem betrachteten Pool (in Bezug auf die Häufigkeit desjenigen Gens in dem betrachteten Pool, das häufiger auftritt): reldiff = |VH - VL| / max(VH, VL), wobei max(VH, VL) = VH für VH > VL und max(VH, VL) = VL für VL ≥ VH

- die Zahl der Selektionszyklen (Ebenen), die vor dem betrachteten Selektionszyklus durchlaufen worden sind: prevnum

- normalisierte Häufigkeit der $V_H$-Gene in dem betrachteten Pool: rpmVH = (VH / sumVH) * 1000000, wobei sumVH die Summe der Häufigkeiten VH aller $V_H$-Gene in dem betrachteten Pool ist

- normalisierte Häufigkeit der $V_L$-Gene in dem betrachteten Pool: rpmVL = (VL / sumVL) * 1000000, wobei sumVL die Summe der Häufigkeiten VL aller $V_L$-Gene in dem betrachteten Pool ist

- normalisierte Häufigkeit der $V_H$-Gene in dem vorangegangenen Pool: prevRpmVH = (prevVH / prevSumVH) * 1000000, wobei prevSumVH die Summe der Häufigkeiten VH aller $V_H$-Gene in dem vorangegangenen Pool ist

- normalisierte Häufigkeit der $V_L$-Gene in dem vorangegangenen Pool: prevRpmVL = (prevVL / prevSumVL) * 1000000, wobei prevSumVL die Summe der Häufigkeiten VL aller $V_L$-Gene in dem vorangegangenen Pool ist

- die relative Veränderung der Zahl der $V_H$-Gene vom ersten Selektionszyklus zum zweiten Selektionszyklus: prevRelDiff$_H$ = (|VH - prevVH|) / max(VH, prevVH), wobei max(VH, prevVH) = VH für VH > prevVH und max(VH, prevVH) = prevVH für prevVH ≥ VH

- die relative Veränderung der Zahl der $V_L$-Gene vom ersten Selektionszyklus zum zweiten Selektionszyklus: prevRelDiff$_L$ = (|VL - prevVL|) / max(VL, prevVL), wobei max(VL, prevVL) = VL für VL > prevVL und max(VL, prevVL) = prevVL für prevVL ≥ VL

- der Logarithmus zur Basis 2 des Verhältnisses der normalisierten Häufigkeit der $V_L$-Gene in dem betrachteten Pool zur normalisierten Häufigkeit der $V_H$-Gene in dem vorangegangenen Pool:

$$logRpmVH = log2(rpmVH/prevRpmsVH)$$

- der Logarithmus zur Basis 2 des Verhältnisses der normalisierten Häufigkeit der $V_L$-Gene in dem betrachteten Pool zur normalisierten Häufigkeit der $V_L$-Gene in dem vorangegangenen Pool:

$$logRpmVL = log2(rpmVL/prevRpmsVL)$$

Es ist denkbar, dass neben den genannten Merkmalen weitere Informationen erfasst/ermittelt werden und in die Erzeugung von Merkmalvektoren einfließen. Beispielsweise können Häufigkeiten von $V_H$-Genen und $V_L$-Genen in Pools aus weiteren Selektionszyklen als Merkmale erfasst werden und in die Erzeugung von Merkmalvektoren einfließen. Ferner können auch Merkmale zur Selektionshierarchie und/oder Parameter der einzelnen Selektionszyklen erfasst werden und in die Erzeugung von Merkmalvektoren einfließen. Dazu zählen beispielsweise auch Informationen über die in den Selektionszyklen verwendeten Substrate, Parameter zur Durchführung der Sequenzierungszyklen (Konzentrationen, Temperaturen, Medien u.a.), Struktur der Sequenzierungshierarchie (Anzahl der Ebenen und/oder Verzweigungen) u.a.

Üblicherweise umfasst der Merkmalvektor eine eindeutige Kennung für das jeweilige $V_L$-Gen und eine eindeutige Kennung für das jeweilige $V_H$-Gen. Die eindeutigen Kennungen dienen der Kennzeichnung des betrachteten $V_H$-Gens und des betrachteten $V_L$-Gens. Eine eindeutige Kennung kann beispielsweise eine Gensequenz, ein Name, eine Kennziffer, ein alphanumerischer Kenncode oder eine andere Kennung sein, die ein $V_L$-Gen bzw. ein $V_H$-Gen eindeutig bezeichnen und damit identifizierbar machen. Die eindeutigen Kennungen dienen daher in erster Linie der Verarbeitung der Ergebnisse und der Zuordnung der Ergebnisse zu den entsprechenden $V_H$- und $V_L$-Genen. Es ist auch denkbar, dass das zu einem Paar gehörige $V_L$-Gen und das zu dem Paar gehörige $V_H$-Gen nicht jeweils einzeln mit einer eindeutigen Kennung versehen werden, sondern das Paar durch eine eindeutige Kennung charakterisiert wird.

Ein Beispiel für einen bevorzugten Merkmalvektor für ein Paar eines $V_L$-Gens und eines $V_H$-Gens mit den jeweiligen Kennungen x und y ist:

<x, y, VL, VH, diff, relDiff, prevVL, prevVH, prevDiff >

Ein Beispiel für einen besonders bevorzugten Merkmalvektor für ein Paar eines $V_L$-Gens und eines $V_H$-Gens mit den jeweiligen Kennungen x und y ist:

<x, y, rpmVL, rpmVH, diff, relDiff, prevDiff, prevRpmVH, prevRpmVL, prevNum, logRpmVH, logRpmVL>

Die Reihenfolgen, in denen die Merkmale in den hier beschriebenen Merkmalvektoren auftreten, können natürlich auch andere sein als die hier dargestellten.

In einem nächsten Schritt werden in der Menge der Daten, die durch die Merkmalvektoren gebildet werden, Anreicherungsmuster identifiziert. Anders als im Stand der Technik üblich wird also die Anreicherung eines Antikörpers oder eines Antikörperfragments nicht nur auf Basis der Häufigkeiten von Genen in dem letzten Pool bestimmt, sondern auf Basis von Merkmalen aus dem letzten Pool und mindestens einem vorangegangenen Pool. Vorzugsweise werden Merkmalvektoren aus mindestens drei, noch mehr bevorzugt aus mindestens vier aufeinander folgenden Pools verwendet, um Häufigkeitsmuster zu identifizieren.

Anreicherungsmuster werden jeweils für Paare von $V_L$-Genen und $V_H$-Genen identifiziert, wobei das $V_L$-Gen und das $V_H$-Gen eines jeden Paares variable Domänen kodieren, die zum selben Antikörper und/oder Antikörperfragment gehören. Die Anreicherungsmuster zeigen, wie stark sich der Antikörper und/oder das Antikörperfragment mit dem jeweiligen $V_L$-Gen und $V_H$-Gen in dem Selektionsverfahren (entlang der Selektionszyklen von Pool zu Pool) anreichern. Die Anreicherungsmuster zeigen also die Bindungsstärken der jeweiligen Antikörper/Antikörperfragmente zu dem in dem Selektionsverfahren verwendeten Antigen (oder den mehreren in dem Selektionsverfahren verwendeten Antigenen) an: je stärker die Anreicherung desto größer die Bindungsstärke.

Vorzugsweise werden Anreicherungsmuster für Paare von $V_L$-Genen und $V_H$-Genen gegenüber mehreren unterschiedlichen Targets identifiziert, vorzugsweise gegenüber einem oder mehreren murinen Target(s) und einem oder mehreren humanen Target(s) identifiziert.

Für jedes Paar eines $V_L$-Gens und eines $V_H$-Gens wird anschließend auf Basis des Anreicherungsmusters jeweils ein Score-Wert berechnet, der die Anreicherung des entsprechenden Antikörpers / Antikörperfragments quantitativ widerspiegelt. Vorzugsweise werden separate Score-Werte für die Anreicherung gegenüber verschiedenen Targets berechnet (beispielsweise ein Score-Wert für die Anreicherung gegenüber einem murinen Target und ein Score-Werte für die Anreicherung gegenüber einem humanen Target).

Ein Ansatz für die Identifizierung eines Anreicherungsmusters ist die Verfolgung der Häufigkeiten der $V_L$-Gene und $V_H$-Gene von einem Pool des Selektionsverfahrens zu einem nächsten Pool. Erfindungsgemäß werden jedoch nicht nur die Häufigkeiten der $V_L$-Gene und $V_H$-Gene allein, sondern zusätzlich ein oder mehrere der oben genannten weiteren Merkmale betrachtet. Zudem wird nicht nur der letzte Pool betrachtet sondern mindestens zwei, vorzugsweise mindestens drei noch mehr bevorzugt mindestens vier aufeinander folgende Pools.

Für die Identifizierung der Anreicherungsmuster wird vorzugsweise auf Methoden der multivariaten Datenanalyse zurückgegriffen.

Vorzugsweise wird dazu in einem ersten Schritt eine Dimensionsreduzierung vorgenommen. Vorzugsweise werden auf Basis der Merkmalvektoren Faktoren für die Paare von $V_L$-Genen und $V_H$-Genen in einem Pool ermittelt, welche die Eigenschaften der Paare in dem Pool mit einer geringeren Zahl an Variablen beschreiben als die Merkmalvektoren. Ein gängiges Verfahren ist zum Beispiel eine Hauptkomponentenanalyse (engl. *Principal Component Analysis* (PCA)). Denkbar ist jedoch auch ein anderes Verfahren der multivariaten Datenanalyse.

Bei der Hauptkomponentenanalyse werden eine Vielzahl von Variablen durch eine geringere Zahl möglichst aussagekräftiger Linearkombinationen (die "Hauptkomponenten") genähert. Ziel der Hauptkomponentenanalyse ist es, die Datenpunkte in einem p-dimensionalen Raum so in einen q-dimensionalen Unterraum zu projizieren, dass dabei möglichst wenig Information verloren geht und vorliegende Redundanz in Form von Korrelation in den Datenpunkten zusammengefasst wird. Dabei geben die positiven ganzen Zahlen p und q die Dimensionen der jeweiligen Räume an. Mathematisch wird eine Hauptachsentransformation durchgeführt: man minimiert die Korrelation mehrdimensionaler Merkmale durch Überführung in einen Vektorraum mit neuer Basis. Die Hauptachsentransformation lässt sich durch eine orthogonale Matrix angeben, die aus den Eigenvektoren der Kovarianzmatrix gebildet wird. Details sind der umfangreichen Fachliteratur zu diesem Thema zu entnehmen (siehe z.B. H.-F. Eckey et al.: Multivariate Statistik, Gabler Verlag 2002, ISBN-13:978-3-409-11969-6).

Vorzugsweise werden die Variablen der Merkmalvektoren durch eine Anzahl an Faktoren angenähert, die mindestens 70% der Variabilität der ursprünglichen Daten erfassen.

In einer bevorzugten Ausführungsform werden 5 bis 7 Variablen eines Merkmalvektors durch 2 oder 3 Faktoren (z.B. Hauptkomponenten) angenähert.

Auf Basis der Faktoren können in einem nächsten Schritt Score-Werte für Paare von $V_L$-Genen und $V_H$-Genen erzeugt werden. Die Score-Werte werden vorzugsweise über alle Pools (mit einem spezifischen Target, z.B. einem murinen Target oder einem humanen Target) erzeugt, für die Merkmalvektoren für die jeweiligen Paare vorliegen. Dies sind (pro Target) mindestens zwei, vorzugsweise mindestens 3, noch mehr bevorzugt mindestens 4 Merkmalvektoren für jedes Paar.

Die Score-Werte korrelieren mit der Anreicherung der $V_L$-Gene und $V_H$-Gene (bzw. der entsprechenden Antikörper / Antikörperfragmente) infolge des Selektionsverfahrens. Üblicherweise ist der Score-Wert umso größer, je ausgeprägter die Anreicherung ist (positive Korrelation).

Der Score-Wert spiegelt die Entwicklung der Faktoren für ein Paar eines $V_L$-Gens und eines $V_H$-Gens vom ersten (betrachteten) Pool zum letzten (betrachteten) Pool des Selektionsverfahrens wider.

In einer bevorzugten Ausführungsform wird die Summe derjenigen Vektoren berechnet, die sich ergeben, wenn man entlang der Koordinaten der Faktoren (Hauptkomponenten) eines Pools vom ersten bis zum letzten Pool schreitet. Für jeden Schritt von den Koordinaten der Faktoren eines Pools zu den Koordinaten der Faktoren des nächsten Pools ergibt sich ein Vektor. Die Summe aller Vektoren ist ein Summenvektor, der, vom Nullpunkt des Koordinatensystems startend, einen Punkt im Koordinatensystem der Faktoren angibt. Als Score-Wert kann beispielsweise die Länge dieses Vektors angegeben werden. Ebenso ist es denkbar, dass der Score-Wert durch Multiplikation der Koordinaten desjenigen Punktes im Koordinatensystem der Faktoren berechnet wird, den der Summenvektor angibt. Es ist denkbar, dass die Koordinaten vor der Multiplikation gewichtet werden. Es ist beispielsweise denkbar, dass dem ersten Faktor (der ersten Hauptkomponente) ein höheres Gewicht beigemessen werden soll als z.B. dem zweiten Faktor (der zweiten Hauptkomponente). Dementsprechend kann man die Koordinate für den ersten Faktor mit einem höheren Gewicht multiplizieren als die Koordinate des zweiten Faktors. In einer bevorzugten Ausführungsform werden die Koordinaten der einzelnen Faktoren gleich gewichtet.

In einem nächsten Schritt können die Score-Werte in einer Rangliste angeordnet werden. Dazu werden die Score-Werte üblicherweise entsprechend ihrer Größe sortiert, z.B. vom größten bis zum kleinsten Score-Wert oder umgekehrt. Korreliert der Score-Wert positiv mit der Anreicherung zeigen die großen Score-Werte die $V_H$-$V_L$-Paare derjenigen Antikörper / Antikörperfragmente an, die eine besonders ausgeprägte spezifische Anreicherung aufweisen. Diese Antikörper / Antikörperfragmente sind daher für therapeutische, immunologische und/oder diagnostische Zwecke von besonderem Interesse. Die Rangliste wird zum Beispiel mittels eines Bildschirms gegenüber einem Nutzer ausgegeben.

Wie bereits beschrieben können verschiedene Score-Werte für Antikörper und/oder Antikörperfragmente ermittelt werden, zum Beispiel ein Score-Wert für die Anreicherung gegenüber einem murinen Target und ein Score-Wert für die Anreicherung gegenüber einem humanen Target. Für jeden der verschiedenen Score-Werte kann eine Rangliste erzeugt werden.

Die berechneten Score-Werte und/oder Ranglisten können zum Beispiel mittels eines Bildschirms gegenüber einem Nutzer ausgegeben werden.

Die Schritte des erfindungsgemäßen Verfahrens können mit Hilfe eines Computersystems oder mehrerer Computersysteme ausgeführt werden.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Geräte können zur Ausführung der Erfindung genutzt werden.

Die Eingaben in den Computer erfolgen über Eingabemittel wie beispielsweise eine Tastatur, eine Maus, ein Mikrofon, ein Netzwerkanschluss, ein externer Datenspeicher und/oder dergleichen. Ausgaben erfolgen üblicherweise über einen Bildschirm (Monitor), auf einem Drucker, über Lautsprecher und/oder durch Speicherung auf einem Datenspeicher.

Ein System zur Ausführung der vorliegenden Erfindung ist konfiguriert, eine Reihe von Operationen auszuführen. In der vorliegenden Beschreibung wurden die Operationen spezifischen Einheiten zugeordnet: Eingabeeinheit, Merkmalvektorerzeugungseinheit, Anreicherungsanalyseneinheit, Score-Wert-Berechnungseinheit, Ranking-Einheit und Ausgabeeinheit. Es ist aber denkbar, dass die Operationen von einer (einzigen) zentralen Verarbeitungseinheit (engl. *Central Processing Unit* (CPU)) eines Computersystems ausgeführt werden. Denkbar ist ferner, dass die Operationen auf verschiedene Computer(systeme) verteilt sind, wobei ein Computer(system) eine oder mehrere Operationen der genannten Einheiten ausführen kann.

Die Erfindung wird nachstehend anhand von Figuren näher erläutert, ohne die Erfindung auf die in den Figuren gezeigten Merkmale und Merkmalskombinationen beschränken zu wollen.

Es zeigen:

Fig. 1 zeigt ein Beispiel für eine Selektionshierarchie mit drei Ebenen (I, II, III). Fig. 1 ist weiter oben näher beschrieben.

Fig. 2 zeigt schematisch einen Biopanning-Zyklus als Beispiel eines Selektionszyklus.

In einem ersten Schritt wird eine Bibliothek (1) von Phagen (1a, 1b, 1c) bereitgestellt, bei denen Antikörper und/oder Antikörperfragmente (10, 11, 12) als Fusionspolypeptide auf Phagen-Hüllproteinen exprimiert sind.

Zusätzlich wird ein Substrat (2) mit immobilisierten Antigenen und/oder Antigenfragmenten bereitgestellt. Die immobilisierten Antigene und/oder Antigenfragmente weisen Bindungsstellen (20) auf, an denen die Antikörper und/oder Antikörperfragmente (10, 11, 12) der Phagen (1a, 1b, 1c) binden können. Bei den Antigenen/Antigenfragmenten kann es sich um Antigene/Antigenfragmente von Maus und/oder Mensch handeln.

In Schritt A des Biopanning-Zyklus werden die Phagen mit dem Substrat inkubiert.

Dabei kommt es zu Wechselwirkungen zwischen den Antikörpern/Antikörperfragmenten und den Antigenen. Der Antikörper/das Antikörperfragment (1c) passt im vorliegenden Fall genau zur Bindungsstelle (20) des immobilisierten Antigens; die Wechselwirkung und die resultierende Bindung sind vergleichsweise stark (stärker als bei den anderen Antikörpern/Antikörperfragmenten).

In Schritt B des Biopanning-Zyklus werden die nicht oder nur schwach an das Substrat bindenden Phagen separiert (abgewaschen). Es verbleiben die stärker bindenden Phagen.

In Schritt C des Biopanning-Zyklus werden die stärker bindenden Phagen vom Substrat gelöst.

In Schritt D des Biopanning-Zyklus werden die vom Substrat gelösten Phagen vermehrt. Das Ergebnis ist eine neue Phagen-Bibliothek, die erneut einem Substrat ausgesetzt werden kann.

Von einem Teil der vom Substrat gelösten Phagen können die Gene der Antikörper und/oder Antikörperfragmente sequenziert werden (Schritt E). Dabei werden die $V_L$-Gene, die die variablen Domänen von leichten Ketten der Antikörper und/oder Antikörperfragmente kodieren und die $V_H$-Gene, die die variablen Domänen der schweren Ketten der Antikörper und/oder Antikörperfragmente kodieren, und auch ihre Häufigkeiten ermittelt.

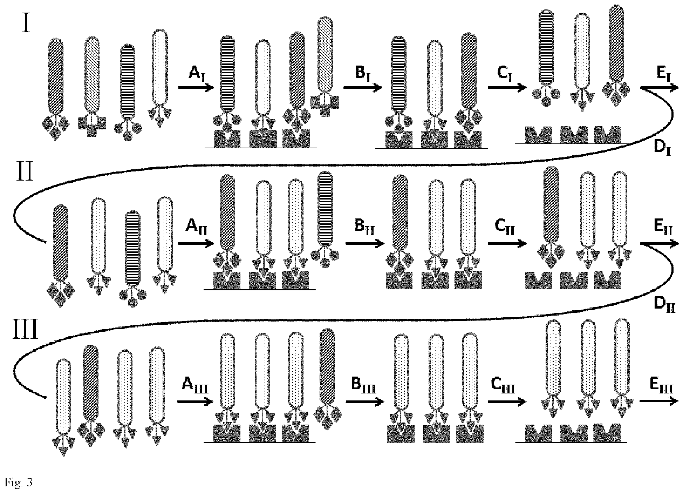

Fig. 3 zeigt schematisch ein Biopanning mit drei Zyklen (I, II, III). Wird wie im vorliegenden Fall jeweils das gleiche Substrat eingesetzt, reichern sich die am stärksten bindenden Antikörper / Antikörperfragmente mit jedem Zyklus weiter an. Die Schritte $A_I$, $B_I$, $C_I$, $D_I$, $E_I$ bzw. $A_{II}$, $B_{II}$, $C_{II}$, $D_{II}$, $E_{II}$ bzw. $A_{III}$, $B_{III}$, $C_{III}$, $D_{III}$, $E_{III}$ entsprechen den Schritten A, B, C, D, E in Fig. 2.

Nach jedem Zyklus (I, II, III) können die $V_H$- und $V_L$-Gene und ihre Häufigkeiten ermittelt werden (Schritte $E_I$, $E_{II}$ und $E_{III}$).

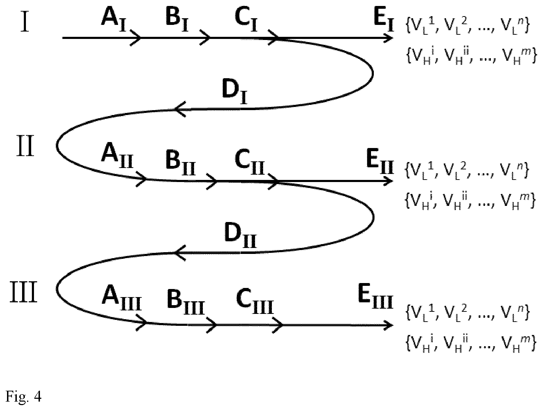

Fig. 4 zeigt schematisch das gleiche Biopanning-Verfahren wie in Fig. 3 mit den Zyklen I, II und III und den Schritten $A_I$, $A_{II}$, $A_{III}$, $B_I$, $B_{II}$, $B_{III}$, $C_I$, $C_{II}$, $C_{III}$, $D_I$, $D_{II}$, $D_{III}$, $E_I$, $E_{II}$ und $E_{III}$. Das Ergebnis der Schritte $E_I$, $E_{II}$ und $E_{III}$ ist jeweils ein Pool an $V_L$-Genen $\{V_L^1, V_L^2, ...,$ $v_L^n\}$ und ein Pool an $V_H$-Genen $\{V_H^i, V_H^{ii}, ..., V_H^m\}$; jedes Gen ist anhand einer eindeutigen Kennung (1, 2, ... bis $n$ bzw. i, ii, ... bis $m$) identifizierbar.

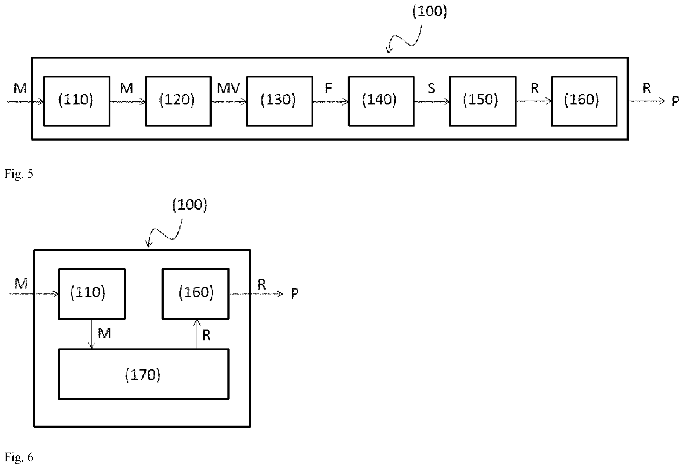

Fig. 5 zeigt schematisch eine Ausführungsform des erfindungsgemäßen Systems. Das System (100) umfasst eine Eingabeeinheit (110), eine Merkmalvektorerzeugungseinheit (120), eine Anreicherungsanalyseneinheit (130), eine Score-Wert-Berechnungseinheit (140), eine Ranking-Einheit (150) und eine Ausgabeeinheit (160).

Über die Eingabeeinheit (110) fließen Merkmale M zu $V_L$-Genen und $V_H$-Genen, die variable Domänen von leichten und schweren Ketten von Antiköpern und/oder Antikörperfragmenten aus einem Selektionsverfahren kodieren, in das System (100) ein. Die Merkmale M werden an die Merkmalvektorerzeugungseinheit (120) übermittelt. Die Merkmalvektorerzeugungseinheit (120) erzeugt auf Basis der Merkmale M Merkmalvektoren MV für Paare von $V_L$- und $V_H$-Genen in den jeweiligen Pools des Selektionsverfahrens, wobei das $V_L$-Gen und das $V_H$-Gen eines jeden Paares variable Domänen kodieren, die zum selben Antikörper und/oder Antikörperfragment gehören. Die Merkmalvektoren MV werden an die Anreicherungsanalyseneinheit (130) übermittelt. Die Anreicherungsanalyseneinheit (130) erzeugt auf Basis der Merkmalvektoren MV Faktoren F, welche die Eigenschaften der $V_L$-$V_H$-Paare in einem Pool mit einer geringeren Zahl an Variablen beschreiben als die Merkmalvektoren. Die Faktoren F werden an die Score-Wert-Berechnungseinheit (140) übermittelt. Die Score-Wert-Berechnungseinheit (140) errechnet auf Basis der Faktoren F Score-Werte S für $V_L$-$V_H$-Paare. Die Score-Werte S werden an die Ranking-Einheit (150) übermittelt. Die Ranking-Einheit (150) erzeugt auf Basis der Score-Werte S eine Rangliste R für $V_L$-$V_H$-Paare. Die Rangliste R wird an die Ausgabeeinheit (160) übermittelt. Die Ausgabeeinheit (160) gibt die Rangliste R gegenüber einem Nutzer P aus.

Das erfindungsgemäße System (100) kann beispielsweise als ein Computersystem (z.B. Desktop-Computer, Tablet-Computer, Smartphone, Server) oder ein Verbund von Computersystemen ausgestaltet sein.

Fig. 6 zeigt schematisch eine weitere Ausführungsform des erfindungsgemäßen Systems. Das System (100) umfasst eine Eingabeeinheit (110), eine Steuer- und Berechnungseinheit (170) und eine Ausgabeeinheit (160).

Die Steuer- und Berechnungseinheit (170) ist konfiguriert:

- die Eingabeeinheit (110) zu veranlassen, Merkmale M zu $V_L$-Genen und $V_H$-Genen zu empfangen, wobei die $V_L$-Gene und $V_H$-Gene die variablen Domänen von leichten und schweren Ketten von Antiköpern und/oder Antikörperfragmenten kodieren, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist.

- Merkmalvektoren für Paare von $V_L$-Genen und $V_H$-Genen in den Pools zu erzeugen, wobei das $V_L$-Gen und das $V_H$-Gen eines jeden Paares variable Domänen kodieren, die zum selben Antikörper und/oder Antikörperfragment gehören,

- Faktoren für die Paare von $V_L$-Genen und $V_H$-Genen in einem Pool auf Basis der Merkmalvektoren zu erzeugen, wobei die Faktoren die Eigenschaften der Paare in einem Pool mit einer geringeren Zahl an Variablen beschreiben als die Merkmalvektoren,

- Score-Werte für Paare von $V_L$-Genen und $V_H$-Genen auf Basis der Faktoren zu berechnen,

- eine Rangliste R für Paare von $V_L$-Genen und $V_H$-Genen auf Basis der Score-Werte zu erzeugen,

- die Ausgabeeinheit (160) zu veranlassen, die Rangliste R gegenüber einem Nutzer P auszugeben.

Das erfindungsgemäße System (100) kann beispielsweise als ein Computersystem (z.B. Desktop-Computer, Tablet-Computer, Smartphone, Server) oder ein Verbund von Computersystemen ausgestaltet sein.

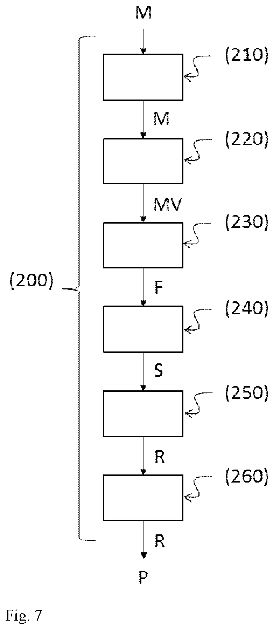

Fig. 7 zeigt in Form eines Ablaufdiagramms eine bevorzugte Ausführungsform des erfindungsgemäßen Verfahrens. Das Verfahren (200) umfasst die Schritte:

(210) Empfangen von Merkmalen M zu $V_L$-Genen und $V_H$-Genen, wobei die $V_L$-Gene und $V_H$-Gene die variablen Domänen von leichten und schweren Ketten von Antiköpern und/oder Antikörperfragmenten kodieren, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,

(220) Bilden von Merkmalvektoren MV für Paare von $V_L$-Genen und $V_H$-Genen in den Pools auf Basis der Merkmale M, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

• Häufigkeit des $V_L$-Gens in dem jeweiligen Pool,
• Häufigkeit des $V_H$-Gens in dem jeweiligen Pool,
• Häufigkeit des $V_L$-Gens in dem vorangegangenen Pool,
• Häufigkeit des $V_H$-Gens in dem vorangegangenen Pool,
• die absolute Differenz zwischen der Häufigkeit des $V_H$-Gens in dem jeweiligen Pool und der Häufigkeit des $V_L$-Gens in dem jeweiligen Pool,
• die absolute Differenz zwischen der Häufigkeit des $V_H$-Gens in dem vorangegangenen Pool und der Häufigkeit des $V_L$-Gens in dem vorangegangenen Pool,

(230) Ermitteln von Faktoren F für die Paare von $V_L$-Genen und $V_H$-Genen in einem Pool auf Basis der Merkmalvektoren MV, wobei die Faktoren F die Eigenschaften der Paare in einem Pool mit einer geringeren Zahl an Variablen beschreiben als die Merkmalvektoren,

(240) Ermitteln von Score-Werten S für Paare von $V_L$-Genen und $V_H$-Genen auf Basis der Faktoren F,

(250) Ermitteln einer Rangliste R für Paare von $V_L$-Genen und $V_H$-Genen auf Basis der Score-Werte S,

(260) Ausgeben der Rangliste R gegenüber einem Nutzer P.

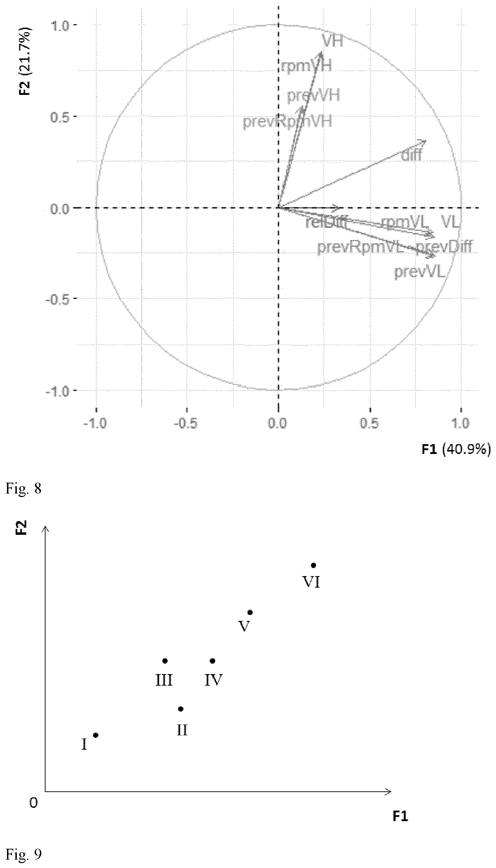

Fig. 8 zeigt beispielhaft in Form einer Grafik das Ergebnis einer Hauptkomponentenanalyse von Merkmalvektoren. F1 ist dabei die erste Hauptkomponente, F2 die zweite Hauptkomponente. In der Grafik ist der Anteil der Variablen der Merkmalvektoren an den Hauptkomponenten F1 und F2 gezeigt. Man erkennt beispielsweise, dass die Anzahl VH der $V_H$-Gene einen großen Anteil an der Hauptkomponente F2 hat, während die Anzahl VL der VL-Gene einen großen Anteil an der Hauptkomponente F1 hat. Mittels der Hauptkomponente F1 lässt sich 40,9% der Variabilität der Daten erklären; mittels Hauptkomponente F2 21,7% der Daten.

Fig. 9 zeigt schematisch die Entwicklung der Werte für die Faktoren eines $V_L$-$V_H$-Paares entlang des Selektionsverfahrens. Das Selektionsverfahren umfasst sechs Selektionszyklen (I, II, II, IV, V, VI), die unmittelbar aufeinander folgen. Das Selektionsverfahren startet also mit dem Selektionszyklus I und endet mit dem Selektionszyklus VI. Für das betrachtete $V_L$-$V_H$-Paar wurden für jeden Pool am Ende eines Selektionszyklus die Merkmalvektoren erzeugt und auf Basis der Merkmalvektoren die Faktoren erzeugt. Fig. 9 zeigt die Variablen der Faktoren F1 und F2 für die einzelnen Pools.

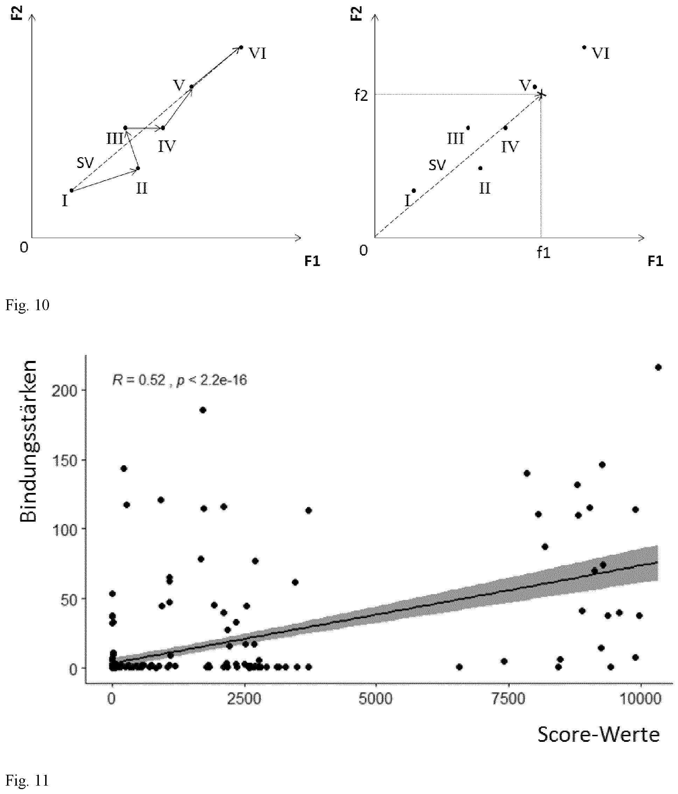

Fig. 10 zeigt schematisch die Erzeugung eines Score-Wertes für ein $V_L$-$V_H$-Paar. Es sind dieselben Daten wie in Fig. 9 gezeigt. Da das Selektionsverfahren entlang der Selektionszyklen I → II → III → IV → V --+ VI verlief, sind entsprechende Vektoren in das Koordinatensystem der Faktoren F1 und F2 eingezeichnet. Die Summe der Vektoren ergibt den gestrichelten Summenvektor SV. Als möglicher Score-Wert für das $V_L$-$V_H$-Paar kann beispielsweise die Länge des Summenvektors SV errechnet werden. Ebenso ist es denkbar, dass als Score-Wert das Produkt f1·f2 der Koordinaten (f, f2) desjenigen Punktes berechnet wird, den der Summenvektor SV angibt, wenn man den Anfang des Vektors in den Ursprung des Koordinatensystems legt. Es ist denkbar, dass die Summanden in dem Summenvektor vor ihrer Addition gewichtet werden. Durch eine solche Wichtung können beispielsweise Selektionszyklen, die zu einer höheren Anreicherung der Antikörper und/oder Antikörperfragmente führen, höher gewichtet werden als Selektionszyklen, die zu einer geringeren Anreicherung führen.

Der Score-Wert dient der Quantifizierung der Bewegung der Variablen der Faktoren vom Ursprung des Koordinatensystems in Richtung höherer Werte für einen oder mehrere Faktoren entlang der Selektionshierarchie. Der beschriebene (gewichtete) Summenvektor quantifiziert beispielsweise die Bewegung der Punkte I bis VI in Richtung höherer Werte für F1 und F2. Andere Score-Werte, die eine solche Bewegung quantifizieren, sind denkbar.

Fig. 11 zeigt beispielhaft die Gegenüberstellung der Score-Werte für $V_L$-$V_H$-Paare mit den Bindungsstärken der entsprechenden Antikörper / Antikörperfragmente, die in einem Enzyme-linked Immunosorbent Assay (ELISA) bestimmt worden sind. Die Bindungsstärken sind auf der Ordinate aufgetragen, die Score-Werte auf der Abszisse. Die Werte korrelieren positiv miteinander.

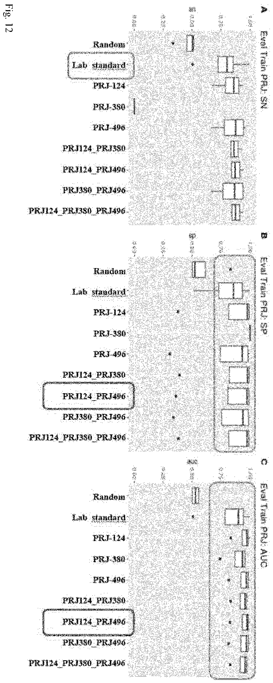

Fig. 12 zeigt einen Vergleich zwischen der Identifizierung von erfolgsversprechenden Antikörpern nach dem erfindungsgemäßen Verfahren und dem üblichen Vorgehen (Auswahl anhand der Häufigkeiten der $V_H$-Gene (auf Basis der $V_H$ CDR3 *counts*)). In Fig. 12 sind drei Performance-Werte grafisch dargestellt; A: die Sensitivität (SN), B: die Spezifizität (SP), C: die so genannte Area under Receiver Operator Characteristic Curve (AUC). Je näher die Werte an dem Wert 1 liegen, desto besser ist die Vorhersage.

Der Eintrag "Random" gibt eine zufällige Auswahl eines Antikörpers wieder. Hier liegen die Werte naturgemäß bei 0,5.

Der Eintrag "Lab standard" zeigt das Ergebnis gemäß üblichem Vorgehen (eine Auswahl von Kandidaten allein auf Basis der $V_H$ CDR3 *counts*).

Die übrigen Einträge zeigen die Ergebnisse der Vorhersage von erfolgversprechenden Kandidaten gemäß der vorliegenden Erfindung für verschiedene Merkmalvektoren an. Alle Vorhersagen sind besser als das übliche Verfahren.

## Identifzieren von $V_L$-$V_H$-Paaren

Die Merkmalvektoren, die bei der Ausführung der vorliegenden Erfindung erzeugt werden, werden für Paare von $V_L$-Genen und $V_H$-Genen in einem Pool erzeugt. Dabei kodieren das $V_L$-Gen und das $V_H$-Gen eines Paares die variablen Domänen von leichten und schweren Ketten, die zum selben Antiköper und/oder Antikörperfragment gehören. Diese Paare werden in dieser Beschreibung auch als $V_L$-$V_H$-Paare bezeichnet. Nachfolgend wird ein Beispiel beschrieben, wie diese Paare identifiziert werden können.

Ausgangspunkt ist ein Selektionsverfahren, das für eine Bibliothek von Antikörpern / Antikörperfragmenten durchgeführt wurde. Die Bibliothek von Antikörpern und/oder Antikörperfragmenten wurde einem Selektionsverfahren zugeführt, um Antikörper und/oder Antikörperfragmente auf Basis ihrer phänotypischen Eigenschaften zu selektieren. Bei dem Selektionsverfahren kann es sich beispielsweise um ein Biopanning-Verfahren handeln. Das Selektionsverfahren umfasst mindestens zwei aufeinander folgende Selektionszyklen, einen ersten Selektionszyklus und einen zweiten Selektionszyklus. Im Anschluss an einen Selektionszyklus werden Gene der selektierten Antikörper und/oder Antikörperfragmente üblicherweise sequenziert. Ziel der Sequenzierung ist die Ermittlung von $V_L$-Genen, die die variablen Domänen von leichten Ketten der Antikörper und/oder Antikörperfragmente kodieren und von $V_H$-Genen, die die variablen Domänen der schweren Ketten der Antikörper und/oder Antikörperfragmente kodieren.

In einem weiteren Schritt des erfindungsgemäßen Verfahrens werden Merkmale zu den $V_L$- und $V_H$-Genen erfasst/ermittelt. Die erfassten Merkmale fließen in einem nachfolgenden Schritt in die Erzeugung von Merkmalvektoren ein.

Folgende Merkmale können in den Merkmalvektor für ein Paar eines $V_L$-Gens und eines $V_H$-Gens aufgenommen werden:

- Information über das betrachtete $V_H$-Gen (z.B. eine eindeutige Kennung)

- Information über das betrachtete $V_L$-Gen (z.B. eine eindeutige Kennung)

- die absolute Zahl VH (Häufigkeit) des betrachteten $V_H$-Gens in dem Pool aus dem zweiten Selektionszyklus

- die absolute Zahl VL (Häufigkeit) des betrachteten $V_L$-Gens in dem Pool aus dem zweiten Selektionszyklus

- die absolute Zahl numVH (Häufigkeit) unterschiedlicher $V_H$-Gene in dem Pool aus dem zweiten Selektionszyklus:

- die absolute Zahl numVL (Häufigkeit) unterschiedlicher $V_L$-Gene in dem Pool aus dem zweiten Selektionszyklus

- die absolute Zahl maxVH (Häufigkeit) desjenigen $V_H$-Gens, das in dem Pool aus dem zweiten Selektionszyklus am häufigsten vorhanden ist

- die absolute Zahl maxVL (Häufigkeit) desjenigen

$V_L$-Gens, das in dem Pool aus dem zweiten Selektionszyklus am häufigsten vorhanden ist

- die relative Häufigkeit relVH des $V_H$-Gens in dem Pool aus dem zweiten Selektionszyklus (bezogen auf die Zahl des am häufigsten in dem Pool aus dem zweiten Selektionszyklus auftretenden $V_H$-Gens): relVH = VH/maxVH

- die relative Häufigkeit relVL des $V_L$-Gens in dem Pool aus dem zweiten Selektionszyklus (bezogen auf die Zahl des am häufigsten in dem Pool aus dem zweiten Selektionszyklus auftretenden $V_L$-Gens): relVL= VL/maxVL

- der Unterschied diff (Abstand) zwischen der Häufigkeit des betrachteten $V_H$-Gens in dem Pool aus dem zweiten Selektionszyklus und der Häufigkeit des betrachteten $V_L$-Gens in dem Pool aus dem zweiten Selektionszyklus (als Betrag): diff = |VH - VL|

- der relative Unterschied reldiff (relativer Abstand) zwischen der Häufigkeit des $V_H$-Gens in dem Pool aus dem zweiten Selektionszyklus und der Häufigkeit des $V_L$-Gens in dem Pool aus dem zweiten Selektionszyklus (in Bezug auf die Häufigkeit desjenigen Gens in dem Pool aus dem zweiten Selektionszyklus, das häufiger auftritt): reldiff = |VH - VL| / max(VH, VL), wobei max(VH, VL) = VH für VH > VL und max(VH, VL) = VL für VL $\geq$ VH

- die Zahl prevnum der Selektionszyklen (Ebenen), die vor dem zweiten Selektionszyklus durchlaufen worden sind

- die absolute Zahl prevVH des $V_H$-Gens in dem Pool aus dem ersten Selektionszyklus

- die absolute Zahl prevVL des $V_L$-Gens in dem Pool aus dem ersten Selektionszyklus

- der Unterschied prevDiff (Abstand) zwischen der Häufigkeit des $V_H$-Gens aus dem Pool des ersten Selektionszyklus und der Häufigkeit des $V_L$-Gens aus dem Pool des ersten Selektionszyklus (als Betrag): prevDiff = |prevVH - prevVL|

- die relative Veränderung der Zahl der $V_H$-Gene vom ersten Selektionszyklus zum zweiten Selektionszyklus: prevRelDiffV$_H$ = (|VH - prevVH|) / max(VH, prevVH), wobei max(VH, prevVH) = VH für VH > prevVH und max(VH, prevVH) = prevVH für prevVH $\geq$ VH

- die relative Veränderung der Zahl der $V_L$-Gene vom ersten Selektionszyklus zum zweiten Selektionszyklus: prevRelDiffV$_L$ = (|VL - prevVL|) / max(VL, prevVL), wobei max(VL, prevVL) = VL für VL > prevVL und max(VL, prevVL) = prevVL für prevVL $\geq$ VL

Es ist denkbar, dass neben den genannten Merkmalen weitere Informationen in die Erzeugung von Merkmalvektoren einfließen.

Die Merkmalvektoren werden einem Modell zugeführt. Das Modell berechnet für jeden Merkmalvektor, der ihm zugeführt wird, ob das Paar eines $V_L$-Gens und eines $V_H$-Gens, dem der Merkmalvektor zugeordnet ist, variable Domänen der leichten und schweren Ketten kodieren, die zum selben Antikörper und/oder Antikörperfragment gehören oder die nicht zum selben Antikörper und/oder Antikörperfragment gehören.

Das Modell kann beispielsweise ein Klassifikationsmodell sein. Ein solches Klassifikationsmodell ordnet jedes Paar von $V_L$- und $V_H$-Genen auf Basis seines Merkmalvektors einer von mindestens zwei Klassen zu. Eine erste Klasse umfasst diejenigen Paare, die variable Domänen der leichten und schweren Ketten kodieren, die zum selben Antikörper und/oder zum selben Antikörperfragment gehören. Eine zweite Klasse umfasst diejenigen Paare, die variable Domänen der leichten und schweren Ketten kodieren, die nicht zum selben Antikörper und/oder nicht zum selben Antikörperfragment gehören.

Vereinfacht ausgedrückt, gibt das Klassifikationsmodell eine Auskunft darüber, ob ein $V_L$-Gen und ein $V_H$-Gen zusammengehören oder nicht. Sie gehören dann zusammen, wenn das $V_L$-Gen die variable Domäne einer leichten Kette eines Antikörpers und/oder Antikörperfragments kodiert, und das $V_H$-Gen die variable Domäne einer schweren Kette desselben Antikörpers und/oder desselben Antikörperfragments kodiert. In einem solchen Fall wird das Paar von $V_L$- und $V_H$-Genen auch als $V_L$-$V_H$-Paar bezeichnet. Sie gehören dann nicht zusammen, wenn das $V_L$-Gen die variable Domäne einer leichten Kette eines Antikörpers und/oder Antikörperfragments kodiert, und das $V_H$-Gen die variable Domäne einer schweren Kette eines anderen Antikörpers und/oder eines anderen Antikörperfragments kodiert.

Bei dem Modell kann es sich auch um ein Regressionsmodell handeln. Das Regressionsmodell kann beispielsweise für jedes Paar eines $V_L$-Gens und eines $V_H$-Gens auf Basis seines Merkmalvektors die Wahrscheinlichkeit berechnen, dass das Paar des $V_L$-Gens und des $V_H$-Gens variable Domänen von leichten und schweren Ketten kodieren, die zum selben Antikörper und/oder zum selben Antikörperfragment gehören. Das Ergebnis der Berechnung des Regressionsmodells kann beispielsweise 0 sein, wenn ausgeschlossen ist, dass das Paar des $V_L$-Gens und des $V_H$-Gens variable Domänen von leichten und schweren Ketten kodieren, die zum selben Antikörper und/oder zum selben Antikörperfragment gehören; das Ergebnis kann beispielsweise 1 oder 100% sein, wenn sicher ist, dass das Paar des $V_L$-Gens und des $V_H$-Gens variable Domänen von leichten und schweren Ketten kodieren, die zum selben Antikörper und/oder zum selben Antikörperfragment gehören. Für die meisten Paare eines $V_L$-Gens und eines $V_H$-Gens wird die berechnete Wahrscheinlichkeit zwischen 0 und 1 bzw. 0 und 100% liegen.

Das Modell (z.B. ein Klassifikationsmodell oder ein Regressionsmodell) wird vorzugsweise auf Basis eines selbstlernenden Algorithmus erstellt. Besonders bevorzugt wird das Modell mittels überwachten Lernens erstellt.

Die Erstellung des Modells kann beispielsweise mit bekannten Antikörpern und/oder Antikörperfragmenten oder mit Paaren von $V_L$- und $V_H$-Genen, für die bekannt ist, ob sie zusammengehören oder nicht, durchgeführt werden. Mit diesen Daten (Trainingsdatensatz) kann ein Modell trainiert werden.

Für die Erstellung von Klassifikationsmodellen gibt es eine Vielzahl an Methoden, wie beispielsweise Random Forest oder Gradient Boosting. Für die Erstellung eines Regressionsmodells gibt es ebenfalls eine Vielzahl an Methoden, wie beispielsweise die logistische Regression. Diese und weitere Verfahren zur Klassifikation und Regression sind vielfältig im Stand der Technik beschrieben (siehe z.B. Norman Matloff: Statistical Regression and Classification - From Linear Models to Machine Learning, Texts in Statistical Science, CRC Press 2017, ISBN 978-1-4987-1091-6; Pratap Dangeti, Statistics for Machine Learning, Packt Publishing 2017, ISBN 978-1-78829-575-8).

Das Ergebnis der Modellerstellung ist ein Modell (z.B. ein Klassifikationsmodell oder ein Regressionsmodell), das auch auf $V_L$- und $V_H$-Häufigkeiten unbekannter Antikörper und/oder Antikörperfragmente anwendbar ist. Die Genauigkeit des Modells ist umso höher, je ähnlicher der Trainingsdatensatz und der Testdatensatz sind. Zum Beispiel ist die Genauigkeit höher, wenn die gleichen Substrate (Antigene) für Training und Test verwendet werden, und niedriger, wenn unterschiedliche Substrate verwendet werden.

Für ein beliebiges Paar von $V_L$- und $V_H$-Genen kann also auf Basis des Modells eine Aussage getroffen werden, ob sie (mit einer definierten Wahrscheinlichkeit) zusammengehören oder nicht. Diese Information kann in einem nächsten Schritt ausgegeben werden. Die Ausgabe kann beispielsweise auf einem Bildschirm eines Computerssystems erfolgen. Die Information kann auch über einen Drucker ausgedruckt oder in einem Datenspeicher gespeichert werden.

**Patentansprüche**

1.  Verfahren umfassend die Schritte:

    - Empfangen von Merkmalen zu $V_L$-Genen und $V_H$-Genen, wobei die $V_L$-Gene und $V_H$-Gene die variablen Domänen von leichten und schweren Ketten von Antiköpern und/oder Antikörperfragmenten kodieren, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,

    - Bilden von Merkmalvektoren für Paare von $V_L$-Genen und $V_H$-Genen in den Pools, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

        • Häufigkeit des $V_L$-Gens in dem jeweiligen Pool,
        • Häufigkeit des $V_H$-Gens in dem jeweiligen Pool,
        • Häufigkeit des $V_L$-Gens in dem vorangegangenen Pool,
        • Häufigkeit des $V_H$-Gens in dem vorangegangenen Pool,
        • die absolute Differenz zwischen der Häufigkeit des $V_H$-Gens in dem jeweiligen Pool und der Häufigkeit des $V_L$-Gens in dem jeweiligen Pool,
        • die absolute Differenz zwischen der Häufigkeit des $V_H$-Gens in dem vorangegangenen Pool und der Häufigkeit des $V_L$-Gens in dem vorangegangenen Pool,

    - Identifizieren von Anreicherungsmustern in den Merkmalvektoren der Pools, wobei Anreicherungsmuster zeigen, wie stark sich der Antikörper und/oder das Antikörperfragment mit dem jeweiligen $V_L$-Gen und $V_H$-Gen in dem Selektionsverfahren anreichern,

    - Ermitteln von Score-Werten für Paare von $V_L$-Genen und $V_H$-Genen auf Basis der Anreicherungsmuster, wobei die Score-Werte mit der jeweiligen Stärke der Anreicherung korrelieren, umfassend folgende Schritte:

        ◦ jeweils für Paare von $V_L$-Genen und $V_H$-Genen, ermitteln eines Vektors zwischen einem Startpunkt und einem Endpunkt in dem Vektorraum der durch die Hauptkomponentenanalyse gebildet wird, wobei der Startpunkt die Hauptkomponenten eines Paares für einen ersten Pool des Selektionsverfahrens und der Endpunkt die Hauptkomponenten desselben Paares für einen letzten Pool des Selektionsverfahrens repräsentieren;
        ◦ Berechnen der Länge des Vektors, oder Berechnen der Länge einer Projektion des Vektors auf eine Achse des Vektorraums, oder Verschieben des Startpunkts des Vektors an den Nullpunkt des Vektorraumes und Berechnen des Produkts der optional gewichteten Hauptkomponenten des Endpunkts des Vektors, und Verwenden des Ergebnisses der jeweiligen Berechnung als Score-Wert für ein Paar eines $V_L$-Gens und $V_H$-Gens;

- Optional, ermitteln einer Rangliste für Paare von $V_L$-Genen und $V_H$-Genen auf Basis der Score-Werte;Ausgeben der Score-Werte und/oder der Rangliste gegenüber einem Nutzer.

2. Verfahren gemäß Anspruch 1, wobei das Identifizieren von Anreicherungsmustern die folgenden Teilschritte umfasst:

- Durchführen einer Dimensionsreduktion aller Merkmalvektoren von Paaren von $V_L$-Genen und $V_H$-Genen in mindestens drei aufeinander folgenden Pools und dabei ermitteln von Faktoren für die Paare von $V_L$-Genen und $V_H$-Genen in den jeweiligen Pools,
- Ermitteln von Score-Werten für die Paare von $V_L$-Genen und $V_H$-Genen durch Quantifizieren der Veränderung der Koordinatenwerte der Faktoren entlang aufeinander folgender Pools.

3. Verfahren gemäß einem der Ansprüche 1 oder 2, umfassend die Schritte:

- Empfangen von Merkmalen zu $V_L$-Genen und $V_H$-Genen, wobei die $V_L$-Gene und $V_H$-Gene die variablen Domänen von leichten und schweren Ketten von Antiköpern und/oder Antikörperfragmenten kodieren, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,
- Bilden von Merkmalvektoren für Paare von $V_L$-Genen und $V_H$-Genen in den Pools auf Basis der Merkmale, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

• Häufigkeit des $V_L$-Gens in dem jeweiligen Pool,
• Häufigkeit des $V_H$-Gens in dem jeweiligen Pool,
• Häufigkeit des $V_L$-Gens in dem vorangegangenen Pool,

• Häufigkeit des $V_H$-Gens in dem vorangegangenen Pool,
• die absolute Differenz zwischen der Häufigkeit des $V_H$-Gens in dem jeweiligen Pool und der Häufigkeit des $V_L$-Gens in dem jeweiligen Pool,
• die absolute Differenz zwischen der Häufigkeit des $V_H$-Gens in dem vorangegangenen Pool und der Häufigkeit des $V_L$-Gens in dem vorangegangenen Pool,

- Ermitteln von Faktoren für die Paare von $V_L$-Genen und $V_H$-Genen in einem Pool auf Basis der Merkmalvektoren, wobei die Faktoren die Eigenschaften der Paare in einem Pool mit einer geringeren Zahl an Variablen beschreiben als die Merkmalvektoren,
- Ermitteln von Score-Werten für Paare von $V_L$-Genen und $V_H$-Genen auf Basis der Faktoren,
- optional: Ermitteln einer Rangliste für Paare von $V_L$-Genen und $V_H$-Genen auf Basis der Score-Werte,
- Ausgeben der Score-Werte und/oder der Rangliste gegenüber einem Nutzer.

4. Verfahren gemäß einem der Ansprüche 1 bis 3, umfassend die Schritte:

- Empfangen von Merkmalen zu $V_L$-Genen und $V_H$-Genen, wobei die $V_L$-Gene und $V_H$-Gene die variablen Domänen von leichten und schweren Ketten von Antiköpern und/oder Antikörperfragmenten kodieren, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,
- Bilden von Merkmalvektoren für Paare von $V_L$-Genen und $V_H$-Genen in den Pools auf Basis der Merkmale, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

• Häufigkeit des $V_L$-Gens in dem jeweiligen Pool,
• Häufigkeit des $V_H$-Gens in dem jeweiligen Pool,
• Häufigkeit des $V_L$-Gens in dem vorangegangenen Pool,
• Häufigkeit des $V_H$-Gens in dem vorangegangenen Pool,
• die absolute Differenz zwischen der Häufigkeit des $V_H$-Gens in dem jeweiligen Pool und der Häufigkeit des $V_L$-Gens in dem jeweiligen Pool,
• die absolute Differenz zwischen der Häufigkeit des $V_H$-Gens in dem vorangegangenen Pool und der Häufigkeit des $V_L$-Gens in dem vorangegangenen Pool,

- Durchführen einer Hauptkomponentenanalyse und Ermitteln der Hauptkomponenten für Paare von $V_L$-Genen und $V_H$-Genen in einem Pool auf Basis der jeweiligen Merkmalvektoren,
- Addieren der Koordinaten der Hauptkomponenten jeweils für Paare von $V_L$-Genen und $V_H$-Genen beginnend mit einem ersten Pool des Selektionsverfahrens und endend mit einem letzten Pool des Selektionsverfahrens und Berechnen der Länge des Vektors, der sich durch die Addition der Koordinaten der Hauptkomponenten ergibt, und Verwenden der Länge des Vektors als Score-Wert für ein Paar eines $V_L$-Gens und $V_H$-Gens,
- optional: Ermitteln einer Rangliste für Paare von $V_L$-Genen und $V_H$-Genen auf Basis der Score-Werte,
- Ausgeben der Score-Werte und/oder der Rangliste gegenüber einem Nutzer.

5. Verfahren gemäß einem der Ansprüche 1 bis 4, wobei ein weiteres Merkmal oder mehrere weitere Merkmale ausgewählt aus der folgenden Liste in die Bildung der Merkmalvektoren einfließen:

• die absolute Zahl (Häufigkeit) unterschiedlicher $V_H$-Gene in dem betrachteten Pool: numVH
• die absolute Zahl (Häufigkeit) unterschiedlicher $V_L$-Gene in dem betrachteten Pool: numVL
• die absolute Zahl (Häufigkeit) desjenigen $V_H$-Gens, das in dem betrachteten Pool am häufigsten vorhanden ist: maxVH
• die absolute Zahl (Häufigkeit) desjenigen $V_L$-Gens, das in dem betrachteten Pool am häufigsten vorhanden ist: maxVL
• die relative Häufigkeit des $V_H$-Gens in dem betrachteten Pool (bezogen auf die Zahl des am häufigsten in dem betrachteten Pool auftretenden $V_H$-Gens): relVH = VH/maxVH
• die relative Häufigkeit des $V_L$-Gens in dem betrachteten Pool (bezogen auf die Zahl des am häufigsten in dem betrachteten Pool auftretenden $V_L$-Gens): relVL = VL/maxVL
• der relative Unterschied (relativer Abstand) zwischen der Häufigkeit des $V_H$-Gens in dem betrachteten Pool und der Häufigkeit des $V_L$-Gens in dem betrachteten Pool (in Bezug auf die Häufigkeit desjenigen Gens in dem betrachteten Pool, das häufiger auftritt): reldiff = |VH - VL| / max(VH, VL), wobei max(VH, VL) = VH für VH > VL und max(VH, VL) = VL für VL ≥ VH
• die Zahl der Selektionszyklen (Ebenen), die vor dem betrachteten Selektionszyklus durchlaufen worden sind: prevnum
• normalisierte Häufigkeit der $V_H$-Gene in dem betrachteten Pool: rpmVH = (VH / sumVH) * 1000000, wobei sumVH die Summe der Häufigkeiten VH aller $V_H$-Gene in dem betrachtenen Pool ist
• normalisierte Häufigkeit der $V_L$-Gene in dem betrachteten Pool: rpmVL = (VL / sumVL) * 1000000, wobei sumVL die Summe der Häufigkeiten VL aller $V_L$-Gene in dem betrachtenen Pool ist
• normalisierte Häufigkeit der $V_H$-Gene in dem vorangegangenen Pool: prevRpmVH = (prevVH / prevSumVH) * 1000000, wobei prevSumVH die Summe der Häufigkeiten VH aller $V_H$-Gene in dem vorangegangenen Pool ist
• normalisierte Häufigkeit der $V_L$-Gene in dem vorangegangenen Pool: prevRpmVL = (prevVL / prevSumVL) * 1000000, wobei prevSumVL die Summe der Häufigkeiten VL aller $V_L$-Gene in dem vorangegangenen Pool ist
• die relative Veränderung der Zahl der $V_H$-Gene vom ersten Selektionszyklus zum zweiten Selektionszyklus: prevRelDiffV$_H$ = (|VH - prevVH|) / max(VH, prevVH), wobei max(VH, prevVH) = VH für VH > prevVH und max(VH, prevVH) = prevVH für prevVH ≥ VH
• die relative Veränderung der Zahl der $V_L$-Gene vom ersten Selektionszyklus zum zweiten Selektionszyklus: prevRelDiffV$_L$ = (|VL - prevVL|) / max(VL, prevVL), wobei max(VL, prevVL) = VL für VL > prevVL und max(VL, prevVL) = prevVL für prevVL ≥ VL

6. Verfahren gemäß einem der Ansprüche 1 bis 4, wobei die Merkmalvektoren die folgenden Merkmale umfassen:

• normalisierte Häufigkeit der $V_L$-Gene in dem betrachteten Pool: rpmVL = (VL / sumVL) * 1000000, wobei sumVL die Summe der Häufigkeiten VL aller $V_L$-Gene in dem betrachteten Pool ist
• normalisierte Häufigkeit der $V_H$-Gene in dem betrachteten Pool: rpmVH = (VH / sumVH) * 1000000, wobei sumVH die Summe der Häufigkeiten VH aller $V_H$-Gene in dem betrachteten Pool ist
• die absolute Differenz (diff) zwischen der Häufigkeit des $V_H$-Gens in dem jeweiligen Pool und der Häufigkeit des $V_L$-Gens in dem jeweiligen Pool (diff = |VH - VL|)
• der relative Unterschied (relativer Abstand) zwischen der Häufigkeit des $V_H$-Gens in dem betrachteten Pool und der Häufigkeit des $V_L$-Gens in dem betrachteten Pool (in Bezug auf die Häufigkeit desjenigen Gens in dem betrachteten Pool, das häufiger auftritt): reldiff = |VH - VL| / max(VH, VL), wobei max(VH, VL) = VH für VH > VL und max(VH, VL) = VL für VL ≥ VH

• die absolute Differenz (prevDiff) zwischen der Häufigkeit des $V_H$-Gens in dem vorangegangenen Pool und der Häufigkeit des $V_L$-Gens in dem vorangegangenen Pool (prevDiff = |prevVH - prevVL|).

• normalisierte Häufigkeit der $V_H$-Gene in dem vorangegangenen Pool: prevRpmVH = (prevVH / prevSumVH) * 1000000, wobei prevSumVH die Summe der Häufigkeiten VH aller $V_H$-Gene in dem vorangegangenen Pool ist

• normalisierte Häufigkeit der $V_L$-Gene in dem vorangegangenen Pool: prevRpmVL = (prevVL / prevSumVL) * 1000000, wobei prevSumVL die Summe der Häufigkeiten VL aller $V_L$-Gene in dem vorangegangenen Pool ist

• die Zahl der Selektionszyklen (Ebenen), die vor dem betrachteten Selektionszyklus durchlaufen worden sind: prevnum

• der Logarithmus zur Basis 2 des Verhältnisses der normalisierten Häufigkeit der $V_H$-Gene in dem betrachteten Pool zur normalisierten Häufigkeit der $V_H$-Gene in dem vorangegangenen Pool:

$$logRpmVH = log2(rpmVH/prevRpmsVH)$$

• der Logarithmus zur Basis 2 des Verhältnisses der normalisierten Häufigkeit der $V_L$-Gene in dem betrachteten Pool zur normalisierten Häufigkeit der $V_L$-Gene in dem vorangegangenen Pool:

$$logRpmVL = log2(rpmVL/prevRpmsVL).$$

7. Verfahren gemäß einem der Ansprüche 1 bis 6, wobei für jedes Paar eines $V_L$-Gens und eines $V_H$-Gens mindestens zwei Score-Werte berechnet werden, ein erster Score-Wert für die Anreicherung gegenüber einem murinen Target und ein zweiter Score-Wert für ein humanes Target, wobei optional für jeden der mindestens zwei Score-Werte eine eigene Rangfolge ermittelt wird, wobei die Score-Werte und/oder die Rangfolgen gegenüber einem Nutzer ausgegeben werden.

8. System umfassend:

   eine Eingabeeinheit,
   eine Merkmalvektorerzeugungseinheit,
   eine Anreicherungsanalyseeinheit,
   eine Score-Wert-Berechnungseinheit,
   eine Ranking-Einheit und
   eine Ausgabeeinheit,

   - wobei die Eingabeeinheit konfiguriert ist, Merkmale zu $V_L$-Genen und $V_H$-Genen, die variable Domänen von leichten und schweren Ketten von Antiköpern und/oder Antikörperfragmenten kodieren, zu erfassen, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,

   - wobei die Merkmalvektorerzeugungseinheit konfiguriert ist, auf Basis der erfassten Merkmale Merkmalvektoren für Paare von $V_L$- und $V_H$- Genen in den Pools zu erzeugen, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

     • Häufigkeit des $V_L$-Gens in dem jeweiligen Pool,
     • Häufigkeit des $V_H$-Gens in dem jeweiligen Pool,
     • Häufigkeit des $V_L$-Gens in dem vorangegangenen Pool,
     • Häufigkeit des $V_H$-Gens in dem vorangegangenen Pool,
     • die absolute Differenz zwischen der Häufigkeit des $V_H$-Gens in dem jeweiligen Pool und der Häufigkeit des $V_L$-Gens in dem jeweiligen Pool,
     • die absolute Differenz zwischen der Häufigkeit des $V_H$-Gens in dem vorangegangenen Pool und der Häufigkeit des $V_L$-Gens in dem vorangegangenen Pool,

   - wobei die Anreicherungsanalyseeinheit konfiguriert ist, Anreicherungsmuster in den Merkmalvektoren der Pools zu identifizieren, wobei Anreicherungsmuster zeigen, wie stark sich der Antikörper und/oder das Antikörperfragment mit dem jeweiligen $V_L$-Gen und $V_H$-Gen in dem Selektionsverfahren anreichern,

   - wobei die Score-Wert-Berechnungseinheit konfiguriert ist, Score-Werte für Paare von $V_L$-Genen und $V_H$-Genen auf Basis der Anreicherungsmuster zu berechnen, wobei die Score-Werte mit der jeweiligen Stärke der Anreicherung korrelieren, und ihre Berechnung folgende Schritte umfasst:

∘ jeweils für Paare von $V_L$-Genen und $V_H$-Genen, ermitteln eines Vektors zwischen einem Startpunkt und einem Endpunkt in dem Vektorraum der durch die Hauptkomponentenanalyse gebildet wird, wobei der Startpunkt die Hauptkomponenten eines Paares für einen ersten Pool des Selektionsverfahrens und der Endpunkt die Hauptkomponenten desselben Paares für einen letzten Pool des Selektionsverfahrens repräsentieren;

∘ Berechnen der Länge des Vektors, oder Berechnen der Länge einer Projektion des Vektors auf eine Achse des Vektorraums, oder Verschieben des Startpunkts des Vektors an den Nullpunkt des Vektorraumes und Berechnen des Produkts der optional gewichteten Hauptkomponenten des Endpunkts des Vektors, und Verwenden des Ergebnisses der jeweiligen Berechnung als Score-Wert für ein Paar eines $V_L$-Gens und $V_H$-Gens;

- Optional, ermitteln einer Rangliste für Paare von $V_L$-Genen und Vn-Genen auf Basis der Score-Werte;
- wobei die Ausgabeeinheit konfiguriert ist, die Score-Werte und/oder der Rangliste gegenüber einem Nutzer auszugeben.

9. Computerprogrammprodukt umfassend einen Datenträger, und Programmcode, der auf dem Datenträger gespeichert ist, und der ein Computersystem, in dessen Arbeitsspeicher der Programmcode geladen ist, dazu veranlasst, die folgenden Schritte auszuführen:

- Empfangen von Merkmalen zu $V_L$-Genen und $V_H$-Genen, wobei die $V_L$-Gene und $V_H$-Gene die variablen Domänen von leichten und schweren Ketten von Antiköpern und/oder Antikörperfragmenten kodieren, wobei die Antikörper und/oder Antikörperfragmente aus einem Selektionsverfahren stammen, wobei das Selektionsverfahren mehrere aufeinander folgende Selektionszyklen umfasst, wobei das Ergebnis eines jeden Selektionszyklus ein Pool mit Antikörpern und/oder Antikörperfragmenten ist,
- Bilden von Merkmalvektoren für Paare von $V_L$-Genen und $V_H$-Genen in den Pools, wobei das $V_L$-Gen und das $V_H$-Gen eines jeden Paares variable Domänen kodieren, die zum selben Antikörper und/oder Antikörperfragment gehören, wobei jeder Merkmalvektor für einen Pool zumindest die folgenden Merkmale umfasst:

• Häufigkeit des $V_L$-Gens in dem jeweiligen Pool,
• Häufigkeit des $V_H$-Gens in dem jeweiligen Pool,
• Häufigkeit des $V_L$-Gens in dem vorangegangenen Pool,
• Häufigkeit des $V_H$-Gens in dem vorangegangenen Pool,
• die absolute Differenz zwischen der Häufigkeit des $V_H$-Gens in dem jeweiligen Pool und der Häufigkeit des $V_L$-Gens in dem jeweiligen Pool,
• die absolute Differenz zwischen der Häufigkeit des $V_H$-Gens in dem vorangegangenen Pool und der Häufigkeit des $V_L$-Gens in dem vorangegangenen Pool,

- Identifizieren von Anreicherungsmustern in den Merkmalvektoren der Pools, wobei Anreicherungsmuster zeigen, wie stark sich der Antikörper und/oder das Antikörperfragment mit dem jeweiligen $V_L$-Gen und $V_H$-Gen in dem Selektionsverfahren anreichen,
- Ermitteln von Score-Werten für Paare von $V_L$-Genen und $V_H$-Genen auf Basis der Anreicherungsmuster, wobei die Score-Werte mit der jeweiligen Stärke der Anreicherung korrelieren, umfassend folgende Schritte:

o jeweils für Paare von $V_L$-Genen und $V_H$-Genen, ermitteln eines Vektors zwischen einem Startpunkt und einem Endpunkt in dem Vektorraum der durch die Hauptkomponentenanalyse gebildet wird, wobei der Startpunkt die Hauptkomponenten eines Paares für einen ersten Pool des Selektionsverfahrens und der Endpunkt die Hauptkomponenten desselben Paares für einen letzten Pool des Selektionsverfahrens repräsentieren;

o Berechnen der Länge des Vektors, oder Berechnen der Länge einer Projektion des Vektors auf eine Achse des Vektorraums, oder Verschieben des Startpunkts des Vektors an den Nullpunkt des Vektorraumes und Berechnen des Produkts der optional gewichteten Hauptkomponenten des Endpunkts des Vektors, und Verwenden des Ergebnisses der jeweiligen Berechnung als Score-Wert für ein Paar eines $V_L$-Gens und $V_H$-Gens;

- optional: Ermitteln einer Rangliste für Paare von $V_L$-Genen und $V_H$-Genen auf Basis der Score-Werte,

- Ausgeben der Score-Werte und/oder der Rangliste gegenüber einem Nutzer.

**10.** Computerprogrammprodukt gemäß Anspruch 9, wobei der Programmcode das Computersystem veranlasst, einen oder mehrere Schritte eines in den Ansprüchen 1 bis 7 genannten Verfahrens auszuführen.

**Claims**

**1.** Method comprising the steps of:

- receiving features relating to $V_L$ genes and $V_H$ genes, the $V_L$ genes and $V_H$ genes encoding the variable domains of light and heavy chains of antibodies and/or antibody fragments, the antibodies and/or antibody fragments originating from a selection method, the selection method comprising multiple successive selection cycles, the result of each selection cycle being a pool containing antibodies and/or antibody fragments,

- forming feature vectors for pairs of $V_L$ genes and $V_H$ genes in the pools, the $V_L$ gene and the $V_H$ gene of each pair encoding variable domains belonging to the same antibody and/or antibody fragment, each feature vector for a pool comprising at least the following features:

• count of the $V_L$ gene in the particular pool,
• count of the $V_H$ gene in the particular pool,
• count of the $V_L$ gene in the preceding pool,
• count of the $V_H$ gene in the preceding pool,
• the absolute difference between the count of the $V_H$ gene in the particular pool and the count of the $V_L$ gene in the particular pool,
• the absolute difference between the count of the $V_H$ gene in the preceding pool and the count of the $V_L$ gene in the preceding pool,

- identifying enrichment patterns in the feature vectors of the pools, enrichment patterns showing the strength of enrichment of the antibody and/or antibody fragment with the particular $V_L$ gene and $V_H$ gene in the selection method,

- ascertaining score values for pairs of $V_L$ genes and $V_H$ genes on the basis of the enrichment patterns, the score values correlating with the respective strength of enrichment, comprising the following steps:

◦ for each pair of a $V_L$ gene and a $V_H$ gene: ascertaining a vector between a start point and an end point in the vector space formed by the principal component analysis, the start point representing the principal components of a pair for a first pool of the selection method and the end point representing the principal components of the same pair for a last pool of the selection method;
◦ calculating the length of the vector, or calculating the length of a projection of the vector onto an axis of the vector space, or shifting the start point of the vector to the zero point of the vector space and calculating the product of the optionally weighted principal components of the end point of the vector, and using the result of the respective calculation as a score value for a pair of a $V_L$ gene and $V_H$ gene;

- optionally: ascertaining a ranking list for pairs of $V_L$ genes and $V_H$ genes on the basis of the score values; outputting the score values and/or the ranking list to a user.

**2.** Method according to Claim 1, wherein identifying enrichment patterns comprises the following substeps:

- carrying out a dimension reduction of all feature vectors of pairs of $V_L$ genes and $V_H$ genes in at least three successive pools and, in doing so, ascertaining factors for the pairs of $V_L$ genes and $V_H$ genes in the respective pools,
- ascertaining score values for the pairs of $V_L$ genes and $V_H$ genes by quantifying the change in the coordinate values of the factors along successive pools.

**3.** Method according to either of Claims 1 and 2, comprising the steps of:

- receiving features relating to $V_L$ genes and $V_H$ genes, the $V_L$ genes and $V_H$ genes encoding the variable domains of light and heavy chains of antibodies and/or antibody fragments, the antibodies and/or antibody fragments originating from a selection method, the selection method comprising multiple successive selection cycles, the result of each selection cycle being a pool containing antibodies and/or antibody fragments,
- forming feature vectors for pairs of $V_L$ genes and $V_H$ genes in the pools on the basis of the features, the $V_L$ gene and the $V_H$ gene of each pair encoding variable domains belonging to the same antibody and/or antibody fragment, each feature vector for a pool comprising at least the following features:

• count of the $V_L$ gene in the particular pool,
• count of the $V_H$ gene in the particular pool,

• count of the $V_L$ gene in the preceding pool,
• count of the $V_H$ gene in the preceding pool,
• the absolute difference between the count of the $V_H$ gene in the particular pool and the count of the $V_L$ gene in the particular pool,
• the absolute difference between the count of the $V_H$ gene in the preceding pool and the count of the $V_L$ gene in the preceding pool,

- ascertaining factors for the pairs of $V_L$ genes and $V_H$ genes in a pool on the basis of the feature vectors, the factors describing the properties of the pairs in a pool with a lower number of variables than the feature vectors,
- ascertaining score values for pairs of $V_L$ genes and $V_H$ genes on the basis of the factors,
- optionally: ascertaining a ranking list for pairs of $V_L$ genes and $V_H$ genes on the basis of the score values,
- outputting the score values and/or the ranking list to a user.

4. Method according to any of Claims 1 to 3, comprising the steps of:

- receiving features relating to $V_L$ genes and $V_H$ genes, the $V_L$ genes and $V_H$ genes encoding the variable domains of light and heavy chains of antibodies and/or antibody fragments, the antibodies and/or antibody fragments originating from a selection method, the selection method comprising multiple successive selection cycles, the result of each selection cycle being a pool containing antibodies and/or antibody fragments,
- forming feature vectors for pairs of $V_L$ genes and $V_H$ genes in the pools on the basis of the features, the $V_L$ gene and the $V_H$ gene of each pair encoding variable domains belonging to the same antibody and/or antibody fragment, each feature vector for a pool comprising at least the following features:

• count of the $V_L$ gene in the particular pool,
• count of the $V_H$ gene in the particular pool,
• count of the $V_L$ gene in the preceding pool,
• count of the $V_H$ gene in the preceding pool,
• the absolute difference between the count of the $V_H$ gene in the particular pool and the count of the $V_L$ gene in the particular pool,
• the absolute difference between the count of the $V_H$ gene in the preceding pool and the count of the $V_L$ gene in the preceding pool,

- carrying out a principal component analysis and ascertaining the principal components for pairs of $V_L$ genes and $V_H$ genes in a pool on the basis of the respective feature vectors,
- adding the coordinates of the principal components for each pair of a $V_L$ gene and a $V_H$ gene starting with a first pool of the selection method and ending with a last pool of the selection method and calculating the length of the vector arising by the addition of the coordinates of the principal components, and using the length of the vector as a score value for a pair of a $V_L$ gene and $V_H$ gene,
- optionally: ascertaining a ranking list for pairs of $V_L$ genes and $V_H$ genes on the basis of the score values,
- outputting the score values and/or the ranking list to a user.

5. Method according to any of Claims 1 to 4, wherein one further feature or multiple further features selected from the following list go into the formation of the feature vectors:

• the absolute number (count) of different $V_H$ genes in the observed pool: numVH
• the absolute number (count) of different $V_L$ genes in the observed pool: numVL
• the absolute number (count) of that $V_H$ gene which is present in the observed pool with the greatest count: maxVH
• the absolute number (count) of that $V_L$ gene which is present in the observed pool with the greatest count: maxVL
• the relative count of the $V_H$ gene in the observed pool (based on the number of the $V_H$ gene which occurs in the observed pool with the greatest count): relVH = VH/maxVH
• the relative count of the $V_L$ gene in the observed pool (based on the number of the $V_L$ gene which occurs in the observed pool with the greatest count): relVL = VL/maxVL
• the relative difference (relative distance) between the count of the $V_H$ gene in the observed pool and the count of the $V_L$ gene in the observed pool (in relation to the count of that gene in the observed pool which occurs with a greater count): reldiff = IVH - VL| / max(VH, VL), where max(VH, VL) = VH for VH > VL and max(VH, VL) = VL for VL ≥ VH
• the number of selection cycles (levels) which were passed through before the observed selection cycle: prevnum
• normalized count of the $V_H$ genes in the observed pool: rpmVH = (VH / sumVH) * 1000000, where sumVH is the sum of the counts VH of all $V_H$ genes in the observed pool
• normalized count of the $V_L$ genes in the observed pool: rpmVL = (VL / sumVL) * 1000000, where sumVL is the sum of the counts VL of all

$V_L$ genes in the observed pool

• normalized count of the $V_H$ genes in the preceding pool: prevRpmVH = (prevVH / prevSumVH) * 1000000, where prevSumVH is the sum of the counts VH of all $V_H$ genes in the preceding pool

• normalized count of the $V_L$ genes in the preceding pool: prevRpmVL = (prevVL / prevSumVL) * 1000000, where prevSumVL is the sum of the counts VL of all $V_L$ genes in the preceding pool

• the relative change in the number of $V_H$ genes from the first selection cycle to the second selection cycle: prevRelDiff$V_H$ = (IVH - prevVHI) / max(VH, prevVH), where max(VH, prevVH) = VH for VH > prevVH and max(VH, prevVH) = prevVH for prevVH $\geq$ VH

• the relative change in the number of $V_L$ genes from the first selection cycle to the second selection cycle: prevRelDiff$V_L$ = (|VL - prevVL|) / max(VL, prevVL), where max(VL, prevVL) = VL for VL > prevVL and max(VL, prevVL) = prevVL for prevVL $\geq$ VL

**6.** Method according to any of Claims 1 to 4, wherein the feature vectors comprise the following features:

• normalized count of the $V_L$ genes in the observed pool: rpmVL = (VL / sumVL) * 1000000, where sumVL is the sum of the counts VL of all $V_L$ genes in the observed pool

• normalized count of the $V_H$ genes in the observed pool: rpmVH = (VH / sumVH) * 1000000, where sumVH is the sum of the counts VH of all $V_H$ genes in the observed pool

• the absolute difference (diff) between the count of the $V_H$ gene in the particular pool and the count of the $V_L$ gene in the particular pool (diff = |VH - VL|)

• the relative difference (relative distance) between the count of the $V_H$ gene in the observed pool and the count of the $V_L$ gene in the observed pool (in relation to the count of that gene in the observed pool which occurs with a greater count): reldiff = |VH - VL| / max(VH, VL), where max(VH, VL) = VH for VH > VL and max(VH, VL) = VL for VL $\geq$ VH

• the absolute difference (prevDiff) between the count of the $V_H$ gene in the preceding pool and the count of the $V_L$ gene in the preceding pool (prevDiff = |prevVH - prevVL|).

• normalized count of the $V_H$ genes in the preceding pool: prevRpmVH = (prevVH / prevSumVH) * 1000000, where prevSumVH is the sum of the counts VH of all $V_H$ genes in the preceding pool

• normalized count of the $V_L$ genes in the preceding pool: prevRpmVL = (prevVL / prevSumVL) * 1000000, where prevSumVL is the sum of the counts VL of all $V_L$ genes in the preceding pool

• the number of selection cycles (levels) which were passed through before the observed selection cycle: prevnum

• the logarithm to the base 2 of the ratio of the normalized count of the $V_H$ genes in the observed pool to the normalized count of the $V_H$ genes in the preceding pool: logRpmVH = log2(rpmVH/prevRpmsVH)

• the logarithm to the base 2 of the ratio of the normalized count of the $V_L$ genes in the observed pool to the normalized count of the $V_L$ genes in the preceding pool: logRpmVL = log2(rpmVL/prevRpmsVL).

**7.** Method according to any of Claims 1 to 6, wherein at least two score values are calculated for each pair of a $V_L$ gene and a $V_H$ gene, a first score value for the enrichment with respect to a murine target and a second score value for a human target, optionally each of the at least two score values having its own rank order ascertained, the score values and/or the rank orders being outputted to a user.

**8.** System comprising:

an input unit,
a feature vector generation unit,
an enrichment analysis unit,
a score value calculation unit,
a ranking unit and
an output unit,

- the input unit being configured to acquire features relating to $V_L$ genes and $V_H$ genes encoding variable domains of light and heavy chains of antibodies and/or antibody fragments, the antibodies and/or antibody fragments originating from a selection method, the selection method comprising multiple successive selection cycles, the result of each selection cycle being a pool containing antibodies and/or antibody fragments,

- the feature vector generation unit being configured to generate feature vectors for pairs of $V_L$ and $V_H$ genes in the pools on the basis of the acquired features, the $V_L$ gene and the $V_H$ gene of each pair encoding variable domains belonging to the same antibody and/or antibody fragment, each feature vector for a pool comprising at least the following features:

• count of the $V_L$ gene in the particular pool,

• count of the $V_H$ gene in the particular pool,
• count of the $V_L$ gene in the preceding pool,
• count of the $V_H$ gene in the preceding pool,
• the absolute difference between the count of the $V_H$ gene in the particular pool and the count of the $V_L$ gene in the particular pool,
• the absolute difference between the count of the $V_H$ gene in the preceding pool and the count of the $V_L$ gene in the preceding pool,

- the enrichment analysis unit being configured to identify enrichment patterns in the feature vectors of the pools, enrichment patterns showing the strength of enrichment of the antibody and/or antibody fragment with the particular $V_L$ gene and $V_H$ gene in the selection method,
- the score value calculation unit being configured to calculate score values for pairs of $V_L$ genes and $V_H$ genes on the basis of the enrichment patterns, the score values correlating with the respective strength of enrichment, and the calculation thereof comprising the following steps:

  ○ for each pair of a $V_L$ gene and a $V_H$ gene: ascertaining a vector between a start point and an end point in the vector space formed by the principal component analysis, the start point representing the principal components of a pair for a first pool of the selection method and the end point representing the principal components of the same pair for a last pool of the selection method;
  ○ calculating the length of the vector, or calculating the length of a projection of the vector onto an axis of the vector space, or shifting the start point of the vector to the zero point of the vector space and calculating the product of the optionally weighted principal components of the end point of the vector, and using the result of the respective calculation as a score value for a pair of a $V_L$ gene and $V_H$ gene;

- optionally: ascertaining a ranking list for pairs of $V_L$ genes and $V_H$ genes on the basis of the score values;
- the output unit being configured to output the score values and/or the ranking list to a user.

9. Computer program product comprising a data carrier, and program code which is stored on the data carrier and which prompts a computer system, in the memory of which the program code is loaded, to execute the following steps:

- receiving features relating to $V_L$ genes and $V_H$ genes, the $V_L$ genes and $V_H$ genes encoding the variable domains of light and heavy chains of antibodies and/or antibody fragments, the antibodies and/or antibody fragments originating from a selection method, the selection method comprising multiple successive selection cycles, the result of each selection cycle being a pool containing antibodies and/or antibody fragments,
- forming feature vectors for pairs of $V_L$ genes and $V_H$ genes in the pools, the $V_L$ gene and the $V_H$ gene of each pair encoding variable domains belonging to the same antibody and/or antibody fragment, each feature vector for a pool comprising at least the following features:

  • count of the $V_L$ gene in the particular pool,
  • count of the $V_H$ gene in the particular pool,
  • count of the $V_L$ gene in the preceding pool,
  • count of the $V_H$ gene in the preceding pool,
  • the absolute difference between the count of the $V_H$ gene in the particular pool and the count of the $V_L$ gene in the particular pool,
  • the absolute difference between the count of the $V_H$ gene in the preceding pool and the count of the $V_L$ gene in the preceding pool,

- identifying enrichment patterns in the feature vectors of the pools, enrichment patterns showing the strength of enrichment of the antibody and/or antibody fragment with the particular $V_L$ gene and $V_H$ gene in the selection method,
- ascertaining score values for pairs of $V_L$ genes and $V_H$ genes on the basis of the enrichment patterns, the score values correlating with the respective strength of enrichment, comprising the following steps:

  ○ for each pair of a $V_L$ gene and a $V_H$ gene: ascertaining a vector between a start point and an end point in the vector space formed by the principal component analysis, the start point representing the principal components of a pair for a first pool of the selection method and the end point representing the principal components of the same pair for a last pool of the selection method;
  ○ calculating the length of the vector, or calculating the length of a projection of the vector onto an axis of the vector space, or shifting the start point of the vector to the zero point of the vector space and calculating the product of the optionally weighted principal components of the end point of the vector, and using the result of the respective calculation as a score value for a pair of a $V_L$ gene and $V_H$ gene;

- optionally: ascertaining a ranking list for pairs of $V_L$ genes and $V_H$ genes on the basis of the score values,
- outputting the score values and/or the ranking list to a user.

10. Computer program product according to Claim 9, wherein the program code prompts the computer system to execute one or more steps of a method stated in Claims 1 to 7.

## Revendications

1. Procédé comprenant les étapes :

   - collecte de caractéristiques relatives aux gènes de $V_L$ et gènes de $V_H$, les gènes de $V_L$ et gènes de $V_H$ codant les domaines variables de chaînes légères et chaînes lourdes d'anticorps et/ou de fragments d'anticorps, les anticorps et/ou fragments d'anticorps étant issus d'un procédé de sélection, le procédé de sélection comprenant plusieurs cycles de sélection successifs, le résultat d'un cycle de sélection respectif étant un pool à anticorps et/ou fragments d'anticorps,
   - construction de vecteurs de caractéristiques pour des paires de gènes de $V_L$ et gènes de $V_H$ dans les pools, le gène de $V_L$ et le gène de $V_H$ d'une paire respective codant des domaines variables, qui appartiennent au(x) même(s) anticorps et/ou fragment d'anticorps, chaque vecteur de caractéristiques pour un pool comprenant au moins les caractéristiques suivantes :

     • fréquence du gène de $V_L$ dans le pool respectif,
     • fréquence du gène de $V_H$ dans le pool respectif,
     • fréquence du gène de $V_L$ dans le pool précédent,
     • fréquence du gène de $V_H$ dans le pool précédent,
     • la différence absolue entre la fréquence du gène de $V_H$ dans le pool respectif et la fréquence du gène de $V_L$ dans le pool respectif,
     • la différence absolue entre la fréquence du gène de $V_H$ dans le pool précédent et la fréquence du gène de $V_L$ dans le pool précédent,

   - identification de motifs d'enrichissement dans les vecteurs de caractéristiques des pools, les motifs d'enrichissement montrant à quel degré l'anticorps et/ou le fragment d'anticorps s'enrichissent en le gène de $V_L$ et le gène de $V_H$ respectifs dans le procédé de sélection,
   - détermination de valeurs de score pour des paires de gènes de $V_L$ et gènes de $V_H$ sur la base des motifs d'enrichissement, les valeurs de score étant en corrélation avec le degré respectif de l'enrichissement, comprenant les étapes suivantes :

     ◦ chaque fois pour des paires de gènes de $V_L$ et gènes de $V_H$, détermination d'un vecteur entre un point initial et un point final dans l'espace vectoriel qui est formé par l'analyse de composants principaux, le point intial représentant les composants principaux d'une paire pour un premier pool du procédé de sélection et le point final représentant les composants principaux de la même paire pour un dernier pool du procédé de sélection ;
     ◦ calcul de la longueur du vecteur, ou calcul de la longueur d'une projection du vecteur sur un axe de l'espace vectoriel, ou déplacement du point initial du vecteur au point zéro de l'espace vectoriel et calcul du produit des composants principaux, en option pondérés, du point final du vecteur, et utilisation du résultat du calcul respectif en tant que valeur de score pour une paire d'un gène de $V_L$ et d'un gène de $V_H$ ;

   - en option, détermination d'un classement pour des paires de gènes de $V_L$ et gènes de $V_H$ sur la base des valeurs de score ; délivrance des valeurs de score et/ou du classement à un utilisateur.

2. Procédé selon la revendication 1, dans lequel l'identification de motifs d'enrichissement comprend les étapes partielles suivantes :

   - exécution d'une réduction de dimension de tous les vecteurs de caractéristiques de paires de gènes de $V_L$ et gènes de $V_H$ dans au moins trois pools successifs et en outre détermination de facteurs pour les paires de gènes de $V_L$ et gènes de $V_H$ dans le pool respectif,
   - détermination de valeurs de score pour les paires de gènes de $V_L$ et gènes de $V_H$ par quantification de la modification des valeurs de coordonnées des facteurs le long de pools successifs.

3. Procédé selon l'une quelconque des revendications 1 et 2, comprenant les étapes :

- collecte de caractéristiques relatives aux gènes de $V_L$ et gènes de $V_H$, les gènes de $V_L$ et gènes de $V_H$ codant les domaines variables de chaînes légères et chaînes lourdes d'anticorps et/ou de fragments d'anticorps, les anticorps et/ou fragments d'anticorps étant issus d'un procédé de sélection, le procédé de sélection comprenant plusieurs cycles de sélection successifs, le résultat d'un cycle de sélection respectif étant un pool à anticorps et/ou fragments d'anticorps,
- construction de vecteurs de caractéristiques pour des paires de gènes de $V_L$ et gènes de $V_H$ dans les pools sur la base des caractéristiques, le gène de $V_L$ et le gène de $V_H$ d'une paire respective codant des domaines variables, qui appartiennent au(x) même(s) anticorps et/ou fragment d'anticorps, chaque vecteur de caractéristiques comprenant pour un pool au moins les caractéristiques suivantes :

• fréquence du gène de $V_L$ dans le pool respectif,
• fréquence du gène de $V_H$ dans le pool respectif,
• fréquence du gène de $V_L$ dans le pool précédent,
• fréquence du gène de $V_H$ dans le pool précédent,
• la différence absolue entre la fréquence du gène de $V_H$ dans le pool respectif et la fréquence du gène de $V_L$ dans le pool respectif,
• la différence absolue entre la fréquence du gène de $V_H$ dans le pool précédent et la fréquence du gène de $V_L$ dans le pool précédent,

- détermination de facteurs pour les paires de gènes de $V_L$ et gènes de $V_H$ dans un pool sur la base des vecteurs de caractéristiques, les facteurs décrivant en tant que les facteurs de caractéristiques les propriétés des paires dans un pool avec un nombre relativement faible de variables,
- détermination de valeurs de score pour des paires de gènes de $V_L$ et gènes de $V_H$ sur la base des facteurs,
- en option : détermination d'un classement pour des paires de gènes de $V_L$ et gènes de $V_H$ sur la base des valeurs de score,
- délivrance des valeurs de score et/ou du classement à un utilisateur.

4. Procédé selon l'une quelconque des revendications 1 à 3, comprenant les étapes :

- collecte de caractéristiques relatives aux gènes de $V_L$ et gènes de $V_H$, les gènes de $V_L$ et gènes de $V_H$ codant les domaines variables de chaînes légères et chaînes lourdes d'anticorps et/ou de fragments d'anticorps, les anticorps et/ou fragments d'anticorps étant issus d'un procédé de sélection, le procédé de sélection comprenant plusieurs cycles de sélection successifs, le résultat d'un cycle de sélection respectif étant un pool à anticorps et/ou fragments d'anticorps,
- construction de vecteurs de caractéristiques pour des paires de gènes de $V_L$ et gènes de $V_H$ dans les pools sur la base des caractéristiques, le gène de $V_L$ et le gène de $V_H$ d'une paire respective codant des domaines variables, qui appartiennent au(x) même(s) anticorps et/ou fragment d'anticorps, chaque vecteur de caractéristiques comprenant pour un pool au moins les caractéristiques suivantes :

• fréquence du gène de $V_L$ dans le pool respectif,
• fréquence du gène de $V_H$ dans le pool respectif,
• fréquence du gène de $V_L$ dans le pool précédent,
• fréquence du gène de $V_H$ dans le pool précédent,
• la différence absolue entre la fréquence du gène de $V_H$ dans le pool respectif et la fréquence du gène de $V_L$ dans le pool respectif,
• la différence absolue entre la fréquence du gène de $V_H$ dans le pool précédent et la fréquence du gène de $V_L$ dans le pool précédent,

- exécution d'une analyse de composants principaux et détermination des composants principaux pour des paires de gènes de $V_L$ et gènes de $V_H$ dans un pool sur la base des vecteurs de caractéristiques respectifs,
- addition des coordonnées des composants principaux respectivement pour des paires de gènes de $V_L$ et gènes de $V_H$ en commençant par un premier pool du procédé de sélection et en terminant avec un dernier pool du procédé de sélection et calcul de la longueur du vecteur, qui résulte de l'addition des coordonnées des composants principaux, et utilisation de la longueur du vecteur en tant que valeur de score pour une paire d'un gène de $V_L$ et d'un gène de $V_H$,
- en option : détermination d'un classement pour des paires de gènes de $V_L$ et gènes de $V_H$ sur la base des valeurs de score,
- délivrance des valeurs de score et/ou du classement à un utilisateur.

**5.** Procédé selon l'une quelconque des revendications 1 à 4, dans lequel une caractéristique supplémentaire ou plusieurs caractéristiques supplémentaires choisies dans la liste suivante interviennent dans la construction des vecteurs de caractéristiques :

• le nombre absolu (fréquence) de gènes de $V_H$ différents dans le pool considéré : numVH
• le nombre absolu (fréquence) de gènes de $V_L$ différents dans le pool considéré : numVL
• le nombre absolu (fréquence) du gène de $V_H$ qui est présent le plus fréquemment dans le pool considéré : maxVH
• le nombre absolu (fréquence) du gène de $V_L$ qui est présent le plus fréquemment dans le pool considéré : maxVL
• la fréquence relative du gène de $V_H$ dans le pool considéré (par rapport au nombre du gène de $V_H$ apparaissant le plus fréquemment dans le pool considéré) : relVH = VH/maxVH
• la fréquence relative fréquence du gène de $V_L$ dans le pool considéré (par rapport au nombre du gène de $V_L$ apparaissant le plus fréquemment dans le pool considéré) : relVL = VL/maxVL
• la différence relative (écart relatif) entre la fréquence du gène de $V_H$ dans le pool considéré et la fréquence du gène de $V_L$ dans le pool considéré (eu égard à la fréquence de ce gène qui, dans le pool considéré, apparaît plus fréquemment) : reldiff = |VH - VL| / max(VH, VL), où max(VH, VL) = VH pour VH > VL et max(VH, VL) = VL fur VL $\geq$ VH
• le nombre des cycles de sélection (niveaux) qui ont été parcourus avant le cycle de sélection considéré : prevnum
• fréquence normalisée des gènes de $V_H$ dans le pool considéré : rpmVH = (VH / sumVH) * 1 000 000, sumVH étant la somme des fréquences VH de tous les gènes de $V_H$e dans le pool considéré
• fréquence normalisée des gènes de $V_L$ dans le pool considéré : rpmVL = (VL / sumVL) * 1 000 000, sumVL étant la somme des fréquences VL de tous les gènes de $V_L$ dans le pool considéré
• fréquence normalisée des gènes de $V_H$ dans le pool précédent :
prevRpmVH = (prevVH / prevSumVH) * 1 000 000, prevSumVH étant la somme des fréquences VH de tous les gènes de $V_H$ dans le pool précédent
• fréquence normalisée des gènes de $V_L$ dans le pool précédent :
prevRpmVL = (prevVL / prevSumVL) * 1 000 000, prevSumVL étant la somme des fréquences VL de tous les gènes de $V_L$ dans le pool précédent
• la modification relative du nombre des gènes de $V_H$ du premier cycle de sélection par rapport au deuxième cycle de sélection : prevRelDiffV$_H$ = (|VH - prevVHI) / max(VH, prevVH), où max(VH, prevVH) = VH pour VH > prevVH et max(VH, prevVH) = prevVH pour prevVH $\geq$ VH
• la modification relative du nombre des gènes de $V_L$ du premier cycle de sélection par rapport au deuxième cycle de sélection : prevRelDiffV$_L$ = (|VL - prevVLI) / max(VL, prevVL), où max(VL, prevVL) = VL pour VL > prevVL et max(VL, prevVL) = prevVL pour prevVL $\geq$ VL

**6.** Procédé selon l'une quelconque des revendications 1 à 4, dans lequel les vecteurs de caractéristiques comprennent les caractéristiques suivantes :

• fréquence normalisée des gènes de $V_L$ dans le pool considéré : rpmVL = (VL / sumVL) * 1 000 000, sumVL étant la somme des fréquences VL de tous les gènes de $V_L$ dans le pool considéré
• fréquence normalisée des gènes de $V_H$ dans le pool considéré : rpmVH = (VH / sum VH) * 1 000 000, sum VH étant la somme des fréquences VH de tous les gènes de $V_H$ dans le pool considéré
• la différence absolue (diff) entre la fréquence du gène de $V_H$ dans le pool respectif et la fréquence du gène de $V_L$ dans le pool respectif (diff = |VH - VL|)
• la différence relative (écart relatif) entre la fréquence du gène de $V_H$ dans le pool considéré et la fréquence du gène de $V_L$ dans le pool considéré (eu égard à la fréquence de ce gène qui, dans le pool considéré, apparaît plus fréquemment) : reldiff = |VH - VL| / max(VH, VL), où max(VH, VL) = VH pour VH > VL et max(VH, VL) = VL pour VL $\geq$ VH
• la différence absolue (prevDiff) entre la fréquence du gène de $V_H$ dans le pool précédent et la fréquence du gène de $V_L$ dans le pool précédent (prevDiff = jprevVH - prevVL|).
• fréquence normalisée des gènes de $V_H$ dans le pool précédent :
prevRpmVH = (prevVH / prevSumVH) * 1 000 000, prevSumVH étant la somme des fréquences VH de tous les gènes de $V_H$ dans le pool précédent
• fréquence normalisée des gènes de $V_L$ dans le pool précédent :
prevRpmVL = (prevVL / prevSumVL) * 1 000 000, prevSumVL étant la somme des fréquences VL de tous les gènes de $V_L$ dans le pool précédent

• le nombre des cycles de sélection (niveaux) qui ont été parcourus avant le cycle de sélection considéré : prevnum

• le logarithme à base 2 du rapport de la fréquence normalisée des gènes de $V_H$ dans le pool considéré à la fréquence normalisée des gènes de $V_H$ dans le pool précédent : logRpmVH = log2(rpmVH/prevRpmsVH)

• le logarithme à base 2 du rapport de la fréquence normalisée des gènes de $V_L$ dans le pool considéré à la fréquence normalisée des gènes de $V_L$ dans le pool précédent : logRpmVL = log2(rpmVL/prevRpmsVL).

**7.** Procédé selon l'une quelconque des revendications 1 à 6, dans lequel pour chaque paire d'un gène de $V_L$ et d'un gène de $V_H$ sont calculées au moins deux valeurs de score, une première valeur de score pour l'enrichissement vis-à-vis d'une cible murine et une deuxième valeur de score pour une cible humaine, un ordre de classement propre étant en option déterminé pour chacune desdites au moins deux valeurs de score, les valeurs de score et/ou les ordres de classement étant délivré(e)s à un utilisateur.

**8.** Système comprenant :

une unité d'entrée,
une unité de génération de vecteurs de caractéristiques,
une unité d'analyse d'enrichissement,
une unité de calcul de valeurs de score,
une unité de classement et
une unité de sortie,

- dans lequel l'unité d'entrée est configurée pour recueillir des caractéristiques relatives à des gènes de $V_L$ et gènes de $V_H$ qui codent des domaines variables de chaînes légères et chaînes lourdes d'anticorps et/ou de fragments d'anticorps, les anticorps et/ou fragments d'anticorps étant issus d'un procédé de sélection, le procédé de sélection comprenant plusieurs cycles de sélection successifs, le résultat d'un cycle de sélection respectif étant un pool à anticorps et/ou fragments d'anticorps,
- dans lequel l'unité de génération de vecteurs de caractéristiques est configurée pour engendrer dans les pools, sur la base des caractéristiques recueillies, des vecteurs de caractéristiques pour des paires de gènes de $V_L$ et gènes de $V_H$, le gène de $V_L$ et le gène de $V_H$ d'une paire respective codant des domaines variables, qui appartiennent au(x) même(s) anticorps et/ou fragment d'anticorps, chaque vecteur de caractéristiques comprenant pour un pool au moins les caractéristiques suivantes : fréquence du gène de $V_L$ dans le pool respectif,

• fréquence du gène de $V_H$ dans le pool respectif,
• fréquence du gène de $V_L$ dans le pool précédent,
• fréquence du gène de $V_H$ dans le pool précédent,
• la différence absolue entre la fréquence du gène de $V_H$ dans le pool respectif et la fréquence du gène de $V_L$ dans le pool respectif,
• la différence absolue entre la fréquence du gène de $V_H$ dans le pool précédent et la fréquence du gène de $V_L$ dans le pool précédent,

- dans lequel l'unité d'analyse d'enrichissement est configurée pour identifier des motifs d'enrichissement dans les vecteurs de caractéristiques des pools, les motifs d'enrichissement montrant à quel degré l'anticorps et/ou le fragment d'anticorps s'enrichissent en le gène de $V_L$ et le gène de $V_H$ respectifs dans le procédé de sélection,
- dans lequel l'unité de calcul de valeurs de score est configurée pour calculer des valeurs de score pour des paires de gènes de $V_L$ et gènes de $V_H$ sur la base des motifs d'enrichissement, les valeurs de score étant en corrélation avec le degré respectif de l'enrichissement, et leur calcul comprenant les étapes suivantes :

◦ chaque fois pour des paires de gènes de $V_L$ et gènes de $V_H$, détermination d'un vecteur entre un point initial et un point final dans l'espace vectoriel qui est formé par l'analyse de composants principaux, le point intial représentant les composants principaux d'une paire pour un premier pool du procédé de sélection et le point final représentant les composants principaux de la même paire pour un dernier pool du procédé de sélection ;
◦ calcul de la longueur du vecteur, ou calcul de la longueur d'une projection du vecteur sur un axe de l'espace vectoriel, ou déplacement du point initial du vecteur au point zéro de l'espace vectoriel et calcul du produit des composants principaux, en option pondérés, du point final du vecteur, et utilisation du résultat du calcul respectif en tant que valeur de score pour une paire d'un gène de $V_L$ et d'un gène de $V_H$ ;

- en option, détermination d'un classement pour des paires de gènes de $V_L$ et gènes de $V_H$ sur la base des valeurs de score;
- dans lequel l'unité de sortie est configurée pour délivrer les valeurs de score et/ou le classement à un utilisateur.

9. Produit programme informatique, comprenant un support de données et un code de programmation qui est mis en mémoire sur le support de données et qui amène un système informatique, dans la mémoire de travail duquel est chargé le code de programmation, à exécuter les étapes suivantes :

- collecte de caractéristiques relatives aux gènes de $V_L$ et gènes de $V_H$, les gènes de $V_L$ et gènes de $V_H$ codant les domaines variables de chaînes légères et chaînes lourdes d'anticorps et/ou de fragments d'anticorps, les anticorps et/ou fragments d'anticorps étant issus d'un procédé de sélection, le procédé de sélection comprenant plusieurs cycles de sélection successifs, le résultat d'un cycle de sélection respectif étant un pool à anticorps et/ou fragments d'anticorps,
- construction de vecteurs de caractéristiques pour des paires de gènes de $V_L$ et gènes de $V_H$ dans les pools, le gène de $V_L$ et le gène de $V_H$ d'une paire respective codant des domaines variables, qui appartiennent au(x) même(s) anticorps et/ou fragment d'anticorps, chaque vecteur de caractéristiques pour un pool comprenant au moins les caractéristiques suivantes :

• fréquence du gène de $V_L$ dans le pool respectif,
• fréquence du gène de $V_H$ dans le pool respectif,
• fréquence du gène de $V_L$ dans le pool précédent,
• fréquence du gène de $V_H$ dans le pool précédent,
• la différence absolue entre la fréquence du gène de $V_H$ dans le pool respectif et la fréquence du gène de $V_L$ dans le pool respectif,
• la différence absolue entre la fréquence du gène de $V_H$ dans le pool précédent et la fréquence du gène de $V_L$ dans le pool précédent,

- identification de motifs d'enrichissement dans les vecteurs de caractéristiques des pools, les motifs d'enrichissement montrant à quel degré l'anticorps et/ou le fragment d'anticorps s'enrichissent en le gène de $V_L$ et le gène de $V_H$ respectifs dans le procédé de sélection,
- détermination de valeurs de score pour des paires de gènes de $V_L$ et gènes de $V_H$ sur la base des motifs d'enrichissement, les valeurs de score étant en corrélation avec le degré respectif de l'enrichissement, comprenant les étapes suivantes :

∘ chaque fois pour des paires de gènes de $V_L$ et gènes de $V_H$, détermination d'un vecteur entre un point initial et un point final dans l'espace vectoriel qui est formé par l'analyse de composants principaux, le point intial représentant les composants principaux d'une paire pour un premier pool du procédé de sélection et le point final représentant les composants principaux de la même paire pour un dernier pool du procédé de sélection ;
o calcul de la longueur du vecteur, ou calcul de la longueur d'une projection du vecteur sur un axe de l'espace vectoriel, ou déplacement du point initial du vecteur au point zéro de l'espace vectoriel et calcul du produit des composants principaux, en option pondérés, du point final du vecteur, et utilisation du résultat du calcul respectif en tant que valeur de score pour une paire d'un gène de $V_L$ et d'un gène de $V_H$ ;

- en option : détermination d'un classement pour des paires de gènes de $V_L$ et gènes de $V_H$ sur la base des valeurs de score,
- délivrance des valeurs de score et/ou du classement à un utilisateur.

10. Produit programme informatique selon la revendication 9, dans lequel le code de programmation amène le système informatique à exécuter une ou plusieurs étapes d'un procédé nommé dans les revendications 1 à 7.

**Abbildungen**

Fig. 1

Fig. 2

I

$A_I$ $B_I$ $C_I$ $E_I$

$D_I$

II

$A_{II}$ $B_{II}$ $C_{II}$ $E_{II}$

$D_{II}$

III

$A_{III}$ $B_{III}$ $C_{III}$ $E_{III}$

Fig. 3

Fig. 4

(100)

M → [ (110) ] M → [ (120) ] MV → [ (130) ] F → [ (140) ] S → [ (150) ] R → [ (160) ] R → P

Fig. 5

(100)

M → [ (110) ]          [ (160) ] R → P

[ (170) ]

Fig. 6

M

(210)

M

(220)

MV

(200)

(230)

F

(240)

S

(250)

R

(260)

R

P

Fig. 7

Fig. 8

Fig. 9

Fig. 10

Fig. 11

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2013178370 A1
- WO 0220822 A2

- WO 2019206729 A

**In der Beschreibung aufgeführte Nicht-Patentliteratur**

- **SAI T. REDDY et al.** Monoclonal antibodies isolated without screening by $\alpha$n$\alpha$ylzing the variable-gene repertoire of plasma cells. *Nature Biotechnology,* September 2010, vol. 28 (9), 965-971
- **H.-F. ECKEY et al.** Multivariate Statistik. Gabler Verlag, 2002

- Statistical Regression and Classification - From Linear Models to Machine Learning. **NORMAN MATLOFF.** Texts in Statistical Science. CRC Press, 2017
- **PRATAP DANGETI.** Statistics for Machine Learning. Packt Publishing, 2017